# EUROPÄISCHE PATENTSCHRIFT

(11) **EP 2 140 840 B1**
(45) Veröffentlichungstag und Bekanntmachung des Hinweises auf die Patenterteilung: **12.09.2012**
(21) Anmeldenummer: 09164381.7
(22) Anmeldetag: 02.07.2009
(51) Int. Cl.: A61F 2/44

(54) **Bandscheibenprothesensystem**
Spinal disc prosthesis system
Système de prothèse de disques intervertébraux

(30) Priorität: 03.07.2008 DE 102008032691
(43) Veröffentlichungstag der Anmeldung: 06.01.2010
(73) Patentinhaber: Aesculap AG, 78532 Tuttlingen (DE)
(72) Erfinder: Beger, Jens, 78532 Tuttlingen (DE); Haas, Alexander, 78166 Donaueschingen (DE)
(74) Vertreter: Hoeger, Stellrecht & Partner Patentanwälte

(56) Entgegenhaltungen:
- WO-A-2007/094923
- US-A1- 2007 191 958
- US-A1- 2007 233 255

## Beschreibung

Die vorliegende Erfindung betrifft ein Bandscheibenprothesensystem zur Ausbildung einer künstlichen Bandscheibe umfassend eine erste Prothesenkomponente und eine von der ersten Prothesenkomponente unabhängige zweite Prothesenkomponente, wobei jede der beiden Prothesenkomponenten ein erstes und ein zweites Wirbelkörperanlageelement zum Anlegen an benachbarte, ein Bandscheibenfach einer Wirbelsäule begrenzende Wirbelkörper und ein relativ zu mindestens einem der ersten und zweiten Wirbelkörperanlageelemente beweglich gelagertes Gelenkelement umfasst, wobei jede Prothesenkomponente ein zwischen den ersten und zweiten Wirbelkörperanlageelementen ausgebildetes Kugelgelenk umfasst.

Ein solches Bandscheibenprothesensystem zur Ausbildung einer künstlichen Bandscheibe umfassend eine erste Prothesenkomponente und eine von der ersten Prothesenkomponente unabhängige zweite Prothesenkomponente, wobei jede der beiden Prothesenkomponenten ein erstes und ein zweites Wirbelkörperanlageelement zum Anlegen an benachbarte, ein Bandscheibenfach einer Wirbelsäule begrenzende Wirbelkörper und ein relativ zu mindestens einem der ersten und zweiten Wirbelkörperanlageelemente beweglich gelagertes Gelenkelement umfasst, ist beispielsweise aus der US 2007/0191958 A1 bekannt. Vorteil derartiger Bandscheibenprothesensysteme ist, dass sie über einen posterioren Zugang in den Patientenkörper implantiert werden können. Anders als bei nur eine einzige Prothesenkomponente umfassenden Bandscheibenprothesen, welche heutzutage standardmäßig über einen anterioren Zugang implantiert werden, müssen beim posterioren Zugang keine großen Gefäße mobilisiert werden, um das Bandscheibenfach zu erreichen. Dadurch können Verletzungen der Gefäße verhindert und eine eventuell erforderliche Revision des Bandscheibenprothesensystems vereinfacht werden. Ferner hat ein posteriorer Zugang den Vorteil, dass gleichzeitig eine häufig erforderliche Dekompression des Rückenmarks oder der Nervenwurzeln im Rahmen des chirurgischen Eingriffs erfolgen kann. Außerdem kann gleichzeitig auch eine eventuell vorhandene Facettenarthrose von dorsal behandelt werden.

Ein Problem bei der Implantation von Bandscheibenprothesensystemen der eingangs beschriebenen Art über einen posterioren Zugang ist die korrekte Platzierung der Prothesenkomponenten. Eine weitere Herausforderung stellt die dabei notwendige Distraktion des Bandscheibenraumes dar, welcher auch als Bandscheibenfach bezeichnet wird. Ein Zugang hierzu ist nur durch die Retraktion der Dura oder der Nervenwurzel möglich. Eine direkte Sicht auf das Bandscheibenfach ist nicht möglich, so dass die Resektion der Bandscheibe und die Platzierung der Prothesenkomponenten längs gekrümmter Wege und für den Operateur quasi "blind" erfolgen muss. Werden zwei Prothesenkomponenten unabhängig voneinander in das Bandscheibenfach eingesetzt, die dann auch nicht miteinander direkt verbunden werden, sondern nur indirekt über die an das Bandscheibenfach angrenzenden Wirbelkörper, so ist es wichtig, dass die Prothesenkomponenten in genau vorgegebener Weise relativ zueinander positioniert werden. Dies ist insbesondere erforderlich, um einen möglichst physiologischen Bewegungsbereich, den sogenannten "Range of Motion" (ROM) auch für das Bandscheibenprothesensystem zu erreichen.

Des Weiteren ist aus der WO 2007/094923 A2 ein Bandscheibenprothesensystem der eingangs beschriebenen Art bekannt.

Es ist daher wünschenswert und somit eine Aufgabe der vorliegenden Erfindung, ein Bandscheibenprothesensystem der eingangs beschriebenen Art so zu verbessern, dass ein im Wesentlichen natürlicher Bewegungsbereich des Bandscheibenprothesensystems möglichst unabhängig von einer Positionierung der beiden Prothesenkomponenten relativ zueinander und zu den benachbarten Wirbelkörpern erreichbar ist.

Diese Aufgabe wird bei einem Bandscheibenprothesensystem der eingangs beschriebenen Art erfindungsgemäß dadurch gelöst, dass ferner eine Führungseinrichtung zum Definieren eines übergeordneten, durch beide Prothesenkomponenten gemeinsam definierten Drehgelenks zum gleichzeitigen Verdrehen der beiden ersten Wirbelkörperanlageelemente relativ zu den beiden zweiten Wirbelkörperanlageelementen um ein gemeinsames Drehzentrum vorgesehen ist, welches in einem Raumbereich zwischen den Mittelpunkten der beiden Kugelgelenke liegt.

Ein derartiges Bandscheibenprothesensystem ermöglicht es, unabhängig von einer genauen Positionierung sowie Orientierung der Prothesenkomponenten eine eindeutige Rotationsachse für eine Flexion beziehungsweise Extension der behandelten Bandscheibe vorzugeben. Ferner können derartige Prothesenkomponenten auch auf einfache Weise so ausgebildet werden, dass ein gemeinsames Dreh- oder Gelenkzentrum unabhängig von der jeweiligen Positionierung der beiden Prothesenkomponenten ist. Dies wird insbesondere bei einem aus der US 2007/0191958 A1 bekannten Bandscheibenprothesensystem gerade nicht erreicht, da hier keine Gleitpaarungen ausbildende Kugelgelenke vorgesehen sind und Radien der aneinander anliegenden Gelenkflächen gerade nicht korrespondierend ausgebildet sind, so dass nicht nur Rotations-, sondern auch über entsprechende Lager Translationsbewegungen zwischen den aneinander anliegenden Teilen der Prothesenkomponente möglich sind, was bei einem dauerhaften Einsatz des Bandscheibenprothesensystems zu einem erhöhten Verschleiß und damit einer kürzeren Standzeit führt. Des Weiteren hat die vorgeschlagene Weiterbildung den Vorteil, dass ein größerer Flächenkontakt zwischen den aneinander anliegenden Teilen und somit kleinere Flächenpressungen erreichbar sind, die die Lebensdauer des Bandscheibenprothesensystems verlängern helfen. Zudem ist die Herstellung eines Kugelgelenks einfach. Die Führungseinrichtung ermöglicht es, unabhängig von einer Orientierung oder Positionierung der Prothesenkomponenten relativ zueinander und relativ zu den das Bandscheibenfach definierenden Wirbelkörpern, ein Drehgelenk auszubilden, welches vorzugsweise nicht nur einen Freiheitsgrad der Rotation, sondern günstigerweise ein Gelenk bildet, welches um beliebig viele Achsen eine Rotation der über die Wirbelkörper relativ zueinander fixierten ersten Wirbelkörperanlageelemente relativ zu den zweiten Wirbelkörperanlageelementen ermöglicht. Die Führungseinrichtung kann insbesondere so ausgebildet sein, dass eine Position des gemeinsamen Drehzentrums ortsfest oder in Abhängigkeit einer Auslenkung des Bandscheibenprothesensystems aus einer Grundstellung variabel ist. Insbesondere kann die Führungseinrichtung einen definierten Verlauf des Drehzentrums im Raum vorgeben, beispielsweise längs einer vorgegebenen Raumkurve, welche zwei oder dreidimensional sein kann. Insbesondere kann das Drehzentrum Rotationen um Achsen definieren, welche von einer Verbindungsgeraden, die die Mittelpunkte der beiden Kugelgelenke miteinander verbindet, abweichen.

Günstigerweise definieren das erste Wirbelkörperanlageelement und das zugeordnete Gelenkelement das jeweilige Kugelgelenk. Diese Ausgestaltung ermöglicht einen besonders kompakten Aufbau der beiden Prothesenkomponenten, insbesondere eine besonders geringe Bauhöhe derselben.

Günstigerweise wird der Raumbereich von zwei senkrecht zu einer Verbindungslinie der beiden Mittelpunkte der beiden Kugelgelenke verlaufenden Ebenen begrenzt. Eine derartige Vorgabe des Raumbereichs ermöglicht einen besonders kompakten Aufbau des Bandscheibenprothesensystems.

Vorteilhafterweise ist das Bandscheibenprothesensystem derart ausgebildet, dass eine Position des Drehzentrums im Raumbereich in definierter Weise in Abhängigkeit einer Auslenkung des Bandscheibenprothesensystems aus einer Grundstellung, in welcher es symmetrisch konfiguriert ist, veränderbar ist. Diese Ausgestaltung des Bandscheibenprothesensystems kommt in ihrer Wirkungsweise einer natürlichen Bandscheibe sehr nahe, bei welcher ein Gelenkzentrum in Abhängigkeit von Relativbewegungen der benachbarten Wirbelkörper zueinander, beispielsweise bei einer Flexion, Extension, einer lateralen Biegung sowie einer Rotation um eine Längsachse der Wirbelsäule oder überlagerte Bewegungen der beschriebenen Art, relativ zu seiner Position in der Grundstellung wandern kann. Insgesamt ist so auf einfache Weise die Ausbildung eines Drehgelenks möglich, mit bewegungsabhängiger translatorischer Komponente einer Position des Gelenkzentrums.

Ein besonders einfache Konstruktion des Bandscheibenprothesensystems kann dadurch erreicht werden, dass eine senkrecht zur Verbindungslinie orientierte Grundstellungsmittelebene zwischen den Mittelpunkten vorgesehen wird, auf welcher das Drehzentrum zumindest in der Grundstellung liegt.

Vorteilhaft ist es, wenn das Bandscheibenprothesensystem derart ausgebildet ist, dass ein Abstand des Drehzentrums von der Grundstellungsmittelebene mit zunehmender Auslenkung des Bandscheibenprothesensystems aus der Grundstellung zunimmt. Eine solche Ausgestaltung ermöglicht die Ausbildung einer künstlichen Bandscheibe, welche in ihrer Funktion einer natürlichen Bandscheibe sehr nahe kommt.

Konstruktiv besonders einfach, ebenso in der Herstellung, wird das Bandscheibenprothesensystem, wenn es derart ausgebildet ist, dass das Drehzentrum unabhängig von einer Auslenkung des Bandscheibenprothesensystems aus der Grundstellung auf der Grundstellungsmittelebene liegt.

Günstigerweise definiert eine Verbindungslinie der Mittelpunkte der beiden Kugelgelenke eine Flexions-/Extensionsachse des Bandscheibenprothesensystems. Diese Verbindungslinie ist insbesondere unabhängig von einer Positionierung der Prothesenkomponenten relativ zueinander.

Um optimale flächige Anlagen der jeweiligen Gelenkflächen aneinander sicherstellen zu können, ist es vorteilhaft, wenn ein Radius der Kugelgelenke größer ist als ein Abstand der beiden Mittelpunkte der Kugelgelenke voneinander.

Besonders einfach wird der Aufbau des Bandscheibenprothesensystems, wenn die erste und die zweite Prothesenkomponente in einer Grundstellung spiegelsymmetrisch zueinander bezogen auf eine zwischen ihnen verlaufende Symmetrie ausgebildet sind. Die spiegelsymmetrische Ausbildung bedeutet nicht, dass die Prothesenkomponenten spiegelsymmetrisch implantiert werden müssen. Durch die spiegelsymmetrische Ausgestaltung kann insbesondere auch erreicht werden, dass laterale Verkippungen der benachbarten Wirbelkörper relativ zueinander, also beispielsweise Rotationen um eine in anterior-posteriorer Richtung verlaufende Drehachse, symmetrisch geführt werden.

Günstigerweise definiert die Verbindungslinie in der Grundstellung eine gemeinsame Rotationsachse des Bandscheibenprothesensystems, welche in lateraler Richtung quer zur Symmetrieachse verläuft. Die gemeinsame Rotationsachse ermöglicht insbesondere eine Flexion/Extension des durch die benachbarten Wirbel in Verbindung mit dem Bandscheibenprothesensystem definierten Wirbelsäulensegments.

Günstig ist es, wenn die Symmetrieebene in anterior-posteriorer Richtung des Bandscheibenprothesensystems verläuft. Insbesondere kann es sich bei der Symmetrieebene, wenn das Bandscheibenprothesensystem implantiert ist, um die Medianebene, also eine durch die Körpermitte gehende Sagittalebene handeln.

Um eine besonders gute Abstützung der benachbarten Wirbelkörper zu erreichen, ist es günstig, wenn Längsachsen der beiden Prothesenkomponenten bezogen auf die Symmetrieebene geneigt sind oder parallel zu dieser verlaufen. In Abhängigkeit insbesondere eines Zustands der benachbarten Wirbelkörper können die beiden Prothesenkomponenten dann optimiert platziert implantiert werden.

Gemäß einer weiteren bevorzugten Ausführungsform der Erfindung kann eine das Drehzentrum enthaltende axiale Rotationsachse vorgesehen sein, welche mindestens in einer unausgelenkten Grundstellung in der Symmetrieebene oder parallel zu dieser und senkrecht oder im Wesentlichen senkrecht zu von den ersten und zweiten Wirbelkörperanlageelementen definierten Anlageflächen verläuft. Unter einer axialen Rotationsachse ist insbesondere eine Rotationsachse zu verstehen, welche eine Rotation um eine von der Wirbelsäule im Bereich des von den benachbarten Wirbelkörpern in Verbindung mit dem Bandscheibenprothesensystem definierte Längsachse des Wirbelsäulensegments definiert.

Auf besonders einfache Weise kann ein Kugelgelenk zwischen dem ersten Wirbelkörperanlageelement und dem Gelenkelement ausgebildet werden, wenn das erste Wirbelkörperanlageelement eine erste Gelenkfläche definiert, wenn das Gelenkelement eine zweite Gelenkfläche definiert, welche korrespondierend zur ersten Gelenkfläche ausgebildet ist, wenn die zweite Gelenkfläche einen Teil einer Kugeloberfläche bildet und wenn die erste Gelenkfläche einen Teil einer Hohlkugel bildet.

Ein besonders einfacher Aufbau der Führungseinrichtung des Bandscheibenprothesensystems kann insbesondere dadurch erreicht werden, dass die Führungseinrichtung an jeder Prothesenkomponente jeweils eine Führungsfläche umfasst zum Führen einer Bewegung der beiden Kugelgelenke jeweils relativ zum zweiten Wirbelkörperanlageelement. Mit anderen Worten bedeutet dies, dass die beiden Kugelgelenke, die insbesondere durch die ersten Wirbelkörperanlageelemente und die Gelenkelemente definiert sein können, relativ zu den zweiten Wirbelkörperanlageelementen in definierter Weise bewegbar sind, um so das gemeinsame Drehzentrum zu definieren. Dabei ist es insbesondere günstig, wenn die Führungseinrichtung sicherstellt, dass die ersten und zweiten Gelenkflächen unabhängig von einer relativen Orientierung der das Bandscheibenfach begrenzenden Wirbelkörper flächig aneinander anliegen.

Mit besonders wenigen Teilen lässt sich das Bandscheibenprothesensystem ausbilden, wenn die Führungsfläche ausgebildet ist zum Führen einer Bewegung des jeweiligen Gelenkelements relativ zum zweiten Wirbelkörperanlageelement. Dies ermöglicht es, prinzipiell die beiden Prothesenkomponenten jeweils aus nur drei Teilen auszubilden, nämlich den beiden Wirbelkörperanlageelementen sowie einem dazwischen und relativ zu beiden Wirbelkörperanlageelementen beweglich gelagerten Gelenkelement.

Vorzugsweise ist die Führungsfläche am Gelenkelement und/oder am zweiten Wirbelkörperanlageelement ausgebildet. Vorzugsweise ist sie so ausgebildet, dass das Gelenkelement und das zweite Wirbelkörperanlageelement unabhängig von einer Relativpositionierung der beiden benachbarten Wirbelkörper flächig aneinander anliegen.

Vorteilhaft ist es, wenn das zweite Wirbelkörperanlageelement eine dritte Gelenkfläche definiert und wenn das Gelenkelement eine vierte Gelenkfläche definiert, welche korrespondierend zur dritten Gelenkfläche ausgebildet ist und wenn die dritte und/oder die vierte Gelenkfläche die Führungsfläche bilden. Diese Ausgestaltung ermöglicht einen besonders kompakten Aufbau des Bandscheibenprothesensystems. Zudem ist so eine besonders gute Führung einer Bewegung des zweiten Wirbelkörperanlageelements und des Gelenkelements relativ zueinander erreichbar.

Günstigerweis definiert die Führungsfläche eine Gelenkebene. Insbesondere kann die Gelenkebene eine Relativbewegung des Gelenkelements und des zweiten Wirbelkörperelements vorgeben, insbesondere durch Einschränken von Bewegungsfreiheitsgraden aus der Gelenkebene heraus oder quer zu dieser.

Um unabhängig von einer Position des Drehzentrums sicherzustellen, dass die ersten und zweiten Gelenkflächen der Kugelgelenke flächig aneinander anliegen, ist es günstig, wenn das Gelenkelement relativ zum zweiten Wirbelkörperanlageelement parallel zur Gelenkebene verschiebbar und/oder um eine senkrecht zur Gelenkebene verlaufende Drehachse verdrehbar ist. Diese Ausgestaltung ermöglicht es, die Mittelpunkte der Kugelgelenke in erforderlicher Weise relativ zueinander zu bewegen, um die Funktionalität des Bandscheibenprothesensystems dauerhaft aufrechtzuerhalten.

Günstig ist es, wenn die Gelenkebene relativ zu einer vom zweiten Wirbelkörperanlageelement definierten, in einer Grundstellung in Richtung auf das erste Wirbelkörperanlageelement weisende Längsachse um einen Neigungswinkel geneigt ist. Durch die Neigung der Gelenkebene kann erreicht werden, dass ein Mittelpunkt des Kugelgelenks relativ zum zweiten Wirbelkörperanlageelement in gewünschter beziehungsweise erforderlicher Weise bewegt werden kann, um auch bei einer Änderung des Drehzentrums insgesamt eine Positionierung der beiden Kugelgelenke an den beiden Prothesenkomponenten derart sicherzustellen, dass eine flächige Anlage der ersten und zweiten Gelenkflächen aneinander ermöglicht wird. Durch die geneigte Gelenkebene werden auf einfache Weise die erforderlichen Freiheitsgrade für die Anpassung der beiden Prothesenkomponenten, unabhängig von einer Positionierung derselben zwischen den benachbarten Wirbelkörpern, bereitgestellt.

Günstige Anpassungen der beiden Prothesenkomponenten relativ zueinander können erreicht werden, wenn der Neigungswinkel einen Wert in einem Bereich von 10° bis 80° aufweist.

Vorzugsweise weist der Neigungswinkel einen Wert in einem Bereich von 30° bis 60° auf.

Besonders vorteilhaft ist es, wenn der Neigungswinkel 45° beträgt. So kann insbesondere eine symmetrische Bewegung des Gelenkelements relativ zum zweiten Wirbelkörperanlageelement vorgegeben werden.

Vorteilhaft ist es, wenn die dritte Gelenkfläche in lateraler Richtung und in Richtung auf das erste Wirbelkörperanlageelement hin weist. Eine solche Gelenkfläche ermöglicht quasi ein Aufgleiten des Gelenkelements am zweiten Wirbelkörperanlageelement bei einer lateralen Beugung der beiden Wirbelkörper relativ zueinander. Insbesondere kann so ein Abstand zwischen den beiden Wirbelkörperanlageelementen unter gleichzeitiger, das heißt abhängiger Querbewegung, in definierter Weise geändert werden. Durch die spezielle Neigung kann das Gelenkelement in Richtung auf das zweite Wirbelkörperanlageelement hin bewegt werden, und zwar bei der Prothesenkomponente, auf deren Seite die Wirbelkörper aufeinander zu bewegt werden. Bei der anderen Prothesenkomponente kann dann ein Aufgleiten des Gelenkelements am zweiten Wirbelkörperanlageelement dahingehend möglich werden, dass ein Abstand zwischen den beiden Wirbelkörperanlageelementen vergrößert wird und durch die abhängige Querbewegung das Gelenkelement etwas in Richtung auf die andere Prothesenkomponente hin verschoben wird.

Gemäß einer weiteren bevorzugten Ausführungsform der Erfindung kann ferner eine Bewegungsbegrenzungseinrichtung vorgesehen sein zum Begrenzen einer Relativbewegung des Gelenkelements und des zweiten Wirbelkörperanlageelements der jeweiligen Prothesenkomponente relativ zueinander. Auf diese Weise kann eine Auslenkung und beispielsweise ein maximaler Neigungswinkel der beiden Wirbelkörper relativ zueinander durch das Bandscheibenprothesensystem begrenzt werden. Insbesondere kann so ein Bewegungsbereich des gemeinsamen Drehzentrums des Bandscheibenprothesensystems definiert und auch begrenzt werden.

Auf besonders einfache Weise kann die Bewegungsbegrenzungseinrichtung ausgebildet werden, wenn sie ein erstes und ein zweites Anschlagglied umfasst, wenn das Gelenkelement das eine der Anschlagglieder und wenn das zweite Wirbelkörperanlageelement das andere der beiden Anschlagglieder aufweist, wenn das erste Anschlagglied einen Flächenbereich der Gelenkebene definiert und wenn das zweite Anschlagglied innerhalb des Flächenbereichs frei beweglich ist. Durch die spezielle Ausgestaltung der beiden Anschlagglieder, die somit zum Begrenzen einer Bewegung des Gelenkelements und des zweiten Wirbelkörperanlageelements zusammenwirken, kann durch die Formgebung des ersten Anschlagglieds sowie des zweiten Anschlagglieds der Flächenbereich definiert werden, innerhalb dessen das Gelenkelement und das zweite Wirbelkörperanlageelement relativ zueinander frei beweglich sind.

Besonders einfach im Aufbau und in der Herstellung wird das Bandscheibenprothesensystem, wenn der Flächenbereich rechteckig oder im Wesentlichen rechteckig ist. Selbstverständlich wäre es auch denkbar, jede beliebige andere Form zu wählen, beispielsweise auch im Querschnitt kreisförmig oder oval. Auch eine eindimensionale Führung ist denkbar, sei es längs einer geradlinigen oder einer gekrümmten oder mehrfach gekrümmten Führungsbahn.

Um auf einfache Weise eine Relativbewegung des Gelenkelements und des zugehörigen zweiten Wirbelkörperanlageelements zu gestatten, ist es vorteilhaft, wenn ein Querschnitt des ersten Anschlagglieds parallel zur Gelenkebene größer ist als ein Querschnitt des zweiten Anschlagglieds parallel zur Gelenkebene. Das zweite Anschlagglied kann dann in das erste Anschlagglied eingeführt werden, hat jedoch mindestens in einer Raumrichtung, vorzugsweise auch in zwei, ausreichend Bewegungsfreiheit, um die gewünschte Relativbewegung zwischen dem Gelenkelement und dem zweiten Wirbelkörperanlageelement zu gestatten. Insbesondere kann es sich dabei um translatorische und rotatorische sowie überlagerte translatorische und rotatorische Bewegungen des Gelenkelements und des zweiten Wirbelkörperanlageelements relativ zueinander handeln.

Günstigerweise ist das zweite Anschlagelement senkrecht oder im Wesentlichen senkrecht zur Gelenkebene orientiert. Diese Ausgestaltung erlaubt einen besonders kompakten Aufbau der Prothesenkomponenten. Ferner ist so sichergestellt, dass eine durch aneinander anschlagende Anschlagglieder wirkende Begrenzungskraft parallel zur Gelenkebene und damit senkrecht zu einer vom zweiten Anschlagglied vorgegebenen Richtung gerichtet ist.

Vorzugsweise ist das erste Anschlagglied in Form einer Ausnehmung ausgebildet und das zweite Anschlagglied in Form eines in die Ausnehmung eintauchenden Vorsprungs. Eine Bewegung der die Anschlagglieder tragenden Bauteile des Bandscheibenprothesensystems ist dann möglich in einem Umfang, den die Anschlagglieder durch ihre Form definieren.

Die Herstellung des Bandscheibenprothesensystems vereinfacht sich weiter, wenn der Vorsprung in Form eines Anschlagstifts ausgebildet ist.

Grundsätzlich denkbar wäre es, den Vorsprung mit einem beliebigen Querschnitt auszubilden. Vorzugsweise weist der Vorsprung einen kreisförmigen Querschnitt auf. Der Vorsprung kann so insbesondere in Form eines zylindrischen Stifts ausgebildet und am Gelenkelement oder am zweiten Wirbelkörperanlageelement angeordnet oder ausgebildet werden.

Vorzugsweise ist ein freies Ende des Vorsprungs halbkugelig geformt. Der Vorsprung kann so insbesondere kantenfrei und korrespondierend zu einem ersten Anschlagglied mit entsprechend verrundeten Innenkanten ausgeformt werden, um möglichst linienförmige Anlagen der Anschlagglieder aneinander zu vermeiden, die zu einem Verkanten der Prothesenteile führen könnten.

Zur Vermeidung eines Verkantens der Anschlagglieder aneinander in Extremstellungen ist es günstig, wenn Innenkanten der Ausnehmung entsprechend dem Querschnitt und/oder einer Form des freien Endes des Vorsprungs gerundet sind. Dies hat ferner den Vorteil, dass sich die Herstellung der Ausnehmung vereinfacht, da sie beispielsweise mit Kugelfräsern hergestellt werden kann.

Die Stabilität des Bandscheibenprothesensystems kann auf einfache Weise dadurch erhöht werden, dass das Gelenkelement das zweite Anschlagglied umfasst. Insbesondere dann, wenn das Gelenkelement kleiner ausgebildet ist als zweite Wirbelkörperanlageelemente, kann das erste Anschlagglied, beispielsweise in Form einer Ausnehmung, idealerweise am zweiten Wirbelkörperanlageelement ausgebildet werden, da häufig dort mehr Platz zur Verfügung steht.

Zur Vereinfachung der Herstellung ist es günstig, wenn das Gelenkelement und das zweite Anschlagglied zweiteilig ausgebildet und unlösbar miteinander verbunden sind. Beispielsweise können sie miteinander verklebt, verschraubt oder verschweißt sein.

Vorteilhafterweise definiert das Gelenkelement eine fünfte Gelenkfläche, welche direkt oder indirekt am zweiten Wirbelkörperanlageelement anliegt. Insbesondere kann das Gelenkelement im Querschnitt im Wesentlichen keilförmig ausgebildet sein, so dass die vierte und fünfte Gelenkfläche Seitenflächen eines Keils definieren, welcher eine weitere Außenfläche in Form der zweiten, kugeligen Gelenkfläche zur Ausbildung des Kugelgelenks aufweist.

Auf einfache Weise lassen sich Aufgleitbewegungen in unterschiedlichen Richtungen realisieren, wenn die fünfte Gelenkfläche relativ zur vierten Gelenkfläche geneigt ist. Ein Neigungswinkel zwischen den Gelenkflächen kann beispielsweise in einem Bereich von 45° bis 135° liegen.

Eine besonders stabile Konfiguration des Bandscheibenprothesensystems kann erreicht werden, wenn eine Schnittlinie der von der vierten und fünften Gelenkfläche definierten Ebenen in anterior-posteriorer Richtung verläuft. Dies gilt insbesondere für eine Grundstellung des Bandscheibenprothesensystems, in welcher es nicht ausgelenkt ist. Vorzugsweise verläuft die Schnittlinie senkrecht oder im Wesentlichen senkrecht zur die Mittelpunkte der Kugelgelenke verbindenden Verbindungsgeraden beziehungsweise Verbindungslinie.

Um eine optimale Abstützung der Gelenkelemente am zweiten Wirbelkörperanlageelement zu erreichen, ist es günstig, wenn die vierte Gelenkfläche in medialer Richtung und wenn die fünfte Gelenkfläche in lateraler Richtung geneigt sind. So kann abhängig von einer Bewegung der benachbarten Wirbelkörper relativ zueinander sichergestellt werden, dass das Gelenkelement stets an der vierten Gelenkfläche anliegt, unabhängig von einem Neigungswinkel der Wirbelkörper relativ zueinander. So kann eine stabile Lagerung erreicht werden und gleichzeitig eine optimale Führung der aneinander anliegenden und geführten Teile der Prothesenkomponenten.

Günstigerweise weist die fünfte Gelenkfläche zwei relativ zueinander geneigte Gelenkflächenbereiche auf, welche relativ zueinander geneigte Gelenkflächenbereichsebenen definieren. Eine solche Ausgestaltung ist insbesondere vorteilhaft, wenn die fünfte Gelenkfläche mit einem Dämpfungs- oder Rückstellglied zusammenwirkt, um eine Bewegung des Gelenkelements in Richtung auf das zweite Wirbelkörperanlageelement hin zu dämpfen. Durch die zwei geneigten Gelenkflächenbereiche können optional unterschiedliche Federkonstanten eines Dämpfungselements angesprochen werden, je nach Richtung der vom Gelenkelement eingeleiteten Kraft.

Um eine zusätzlich gute Führung zu erreichen, ist es günstig, wenn der eine der Gelenkflächenbereiche relativ zu einer vom zweiten Wirbelkörperanlageelement definierten Längsachse stärker geneigt ist als die Gelenkebene und wenn der andere der Gelenkflächenbereiche relativ zur Längsachse weniger stark geneigt ist als die Gelenkebene. Auf diese Weise definieren die beiden Gelenkflächenbereiche Seitenflächen einer keilförmigen Ausnehmung, welche insbesondere für eine Relativbewegung in anterior-posteriorer Richtung eine gute Führung bilden kann.

Günstig ist es, wenn ein Neigungswinkel des einen Gelenkflächenbereichs relativ zur Längsachse in einem Bereich von 40° bis 60° liegt und wenn ein Neigungswinkel des anderen Gelenkflächenbereichs relativ zur Längsachse in einem Bereich von 10° bis 30° liegt. Unter Längsachse ist hier insbesondere die vom zweiten Wirbelkörperanlageelement definierte Längsachse in Richtung auf das erste Wirbelkörperanlageelement hin zu verstehen. Die angegebenen Neigungswinkelbereiche ermöglichen ein einfaches Herstellen des Gelenkelements und helfen zudem, ein Kippen desselben bezüglich des zweiten Wirbelkörperanlageelements zu verhindern.

Besonders einfach wird der Aufbau des zweiten Gelenkelements, wenn eine Schnittlinie der Gelenkflächenbereichsebenen in anterior-posteriorer Richtung verläuft. Dies ermöglicht insbesondere eine optimale Führung bei einer Flexion beziehungsweise Extension der Wirbelsäule.

Gemäß einer bevorzugten Ausführungsform der Erfindung kann ferner eine Rückstelleinrichtung zum Rückführen der beiden Kugelgelenke der Prothesenkomponenten von einer aus einer Grundstellung ausgelenkten Gelenkstellung zurück in die Grundstellung vorgesehen sein. Die Rückstelleinrichtung kann insbesondere auch als Dämpfungseinrichtung dienen, um einer auf das Bandscheibenfach wirkenden, von den beiden angrenzenden Wirbelkörper eingeleiteten Kompressionskraft entgegenzuwirken und ein Aufeinanderschlagen der Teile der Prothesenkomponenten zu vermeiden.

Günstigerweise ist die Rückstelleinrichtung derart ausgebildet, dass in einer Grundstellung des Bandscheibenprothesensystems das zweite Anschlagglied von seitlichen Begrenzungsflächen des ersten Anschlagelements, welches den Flächenbereich definiert, beabstandet ist. Diese Ausgestaltung ermöglicht es, das Gelenkelement frei in allen Richtungen, die der Flächenbereich zulässt, relativ zum zweiten Wirbelkörperanlageelement zu bewegen. Insbesondere ist es dann möglich, ausgehend von der Grundstellung direkt entgegen der Wirkung der Rückstelleinrichtung das Gelenkelement relativ zum zweiten Wirbelkörperelement zu bewegen, beispielsweise infolge der Einleitung einer Kraft über das erste Wirbelkörperanlageelement auf das Gelenkelement.

Vorteilhaft ist es, wenn die Rückstelleinrichtung derart ausgebildet ist, dass in einer Grundstellung des Bandscheibenprothesensystems das Gelenkelement ohne die Rückstelleinrichtung vorzuspannen mit dieser in Kontakt steht. Bei der Grundstellung kann es sich insbesondere um eine noch nicht implantierte Stellung der Teile des Bandscheibenprothesensystems relativ zueinander handeln oder auch um eine implantierte Stellung.

Ein besonders einfacher Aufbau des Bandscheibenprothesensystems kann dadurch erreicht werden, dass die Rückstelleinrichtung an der fünften Gelenkfläche angreift. Insbesondere kann die Rückstelleinrichtung derart ausgebildet sein, dass sie ausschließlich an der fünften Gelenkfläche des Gelenkelements angreift und sich ansonsten am zweiten Wirbelkörperanlageelement abstützt.

Vorzugsweise ist die Rückstelleinrichtung derart ausgebildet, dass in einer Grundstellung des Bandscheibenprothesensystems das zweite Wirbelkörperanlageelement ohne die Rückstelleinrichtung vorzuspannen mit dieser in Kontakt steht. Insbesondere ist es so möglich, eine Prothesenkomponente auszubilden, bei der sich das Gelenkelement einerseits direkt am zweiten Wirbelkörperelement abstützt und andererseits indirekt über die dazwischen geschaltete Rückstelleinrichtung, so dass eine Bewegung parallel zur Gelenkfläche bei entsprechend einwirkenden Kräften gedämpft werden kann.

Optional ist es günstig, wenn die Rückstelleinrichtung in der Grundstellung federnd vorgespannt ist. Die Grundstellung kann dabei wiederum eine implantierte oder eine nicht implantierte Stellung des Bandscheibenprothesensystems vorgeben. Die federnde Vorspannung hat den Vorteil, dass auch noch bei einer Bewegung des Gelenkelements in einer Richtung vom zweiten Wirbelkörperanlageelement weg zunächst noch ein Kontakt zwischen der Rückstelleinrichtung und dem Gelenkelement besteht, es also nicht sofort zu einem Abheben der Teile voneinander kommen kann.

Vorteilhafterweise ist das Gelenkelement aus der Grundstellung entgegen einer Wirkung der Rückstelleinrichtung in lateraler Richtung bewegbar. Diese Ausgestaltung gestattet es, Abweichungen von einer parallelen Ausrichtung der Prothesenkomponenten in anterior-posteriorer Richtung auszugleichen. Insbesondere kann so die Rückstelleinrichtung zusätzlich Einfluss nehmen auf eine Positionierung des Drehzentrums des Bandscheibenprothesensystems und gezielt ein Abheben der Gelenkelemente der Prothesenkomponenten von den jeweiligen zweiten Wirbelkörpern verhindern.

Eine optimale Dämpfung des Bandscheibenprothesensystems in axialer Richtung kann insbesondere dadurch erreicht werden, dass das Gelenkelement aus der Grundstellung entgegen einer Wirkung der Rückstelleinrichtung in Richtung auf das zweite Wirbelkörperanlageelement hin bewegbar ist. Insbesondere eine keilförmige Ausgestaltung des zweiten Gelenkelements kann so zu einer definierten Krafteinleitung führen und ein Aufeinanderschlagen, beispielsweise aus einem Metall hergestellter Teile, der Prothesenkomponenten verhindern.

Insbesondere zum Stabilisieren des das Bandscheibenprothesensystem umfassenden Wirbelsäulensegments ist es vorteilhaft, wenn das Gelenkelement aus der Grundstellung entgegen einer Wirkung der Rückstelleinrichtung in anteriorer Richtung bewegbar ist. So kann insbesondere eine Flexionsbewegung der Wirbelsäule gezielt gedämpft werden.

In analoger Weise ist es vorteilhaft, wenn das Gelenkelement aus der Grundstellung entgegen einer Wirkung der Rückstelleinrichtung in posteriorer Richtung bewegbar ist. So kann auch bei einer Extensionsbewegung des das Bandscheibenprothesensystem umfassenden Wirbelsäulensegments eine gezielte Dämpfung eingestellt und erreicht werden.

Vorteilhaft ist es, wenn die Rückstelleinrichtung mindestens ein Rückstellglied umfasst, welches einerseits am Gelenkelement und andererseits am zweiten Wirbelkörperanlageelement angreift. Das mindestens eine Rückstellglied kann insbesondere so angeordnet sein, dass eine Bewegung eines geführten, am zweiten Wirbelkörperanlageelement anliegenden Gelenkelements vom ersten Wirbelkörperanlageelement weg in Richtung auf das zweite Wirbelkörperanlageelement hin gedämpft wird. Außerdem lässt sich so die Rückstelleinrichtung auf besonders einfache Weise ausbilden.

Insbesondere bei einer bezogen auf eine Symmetrieebene des Bandscheibenprothesensystems geneigte Gelenkfläche ist es vorteilhaft, wenn die Rückstelleinrichtung zwei Rückstellglieder umfasst, welche Kraftrichtungen definieren, die linear unabhängig voneinander sind. Mit anderen Worten bedeutet dies, dass die beiden Kraftrichtungen in jeweils zwei Komponenten zerlegbar sind, wovon nur jeweils eine in die gleiche Richtung oder in zueinander entgegensetzte Richtungen weisen. Insbesondere ist linear unabhängig im mathematischen Sinne zu verstehen.

Besonders einfach wird der Aufbau der Rückstelleinrichtung, wenn sie nur ein einziges Rückstellglied umfasst.

Besonders stabil ausbilden lässt sich das Bandscheibenprothesensystem, wenn das mindestens eine Rückstellglied eine Gelenkelementanlagefläche aufweist, welche in der Grundstellung an der fünften Gelenkfläche anliegt. Das Gelenkelement kann sich so zum einen an der vierten Gelenkfläche des zweiten Wirbelkörperanlageelements und zum anderen am mindestens einen Rückstellglied abstützen, und zwar in der Grundstellung sowie in einer Stellung, in welcher das Rückstellglied entgegen der Wirkung einer durch das Gelenkelement eingeleiteten Kraft komprimiert ist.

Vorteilhafterweise umfasst die Gelenkelementanlagefläche zwei Gelenkelementanlageflächenabschnitte, welche relativ zueinander geneigte Gelenkelementanlageflächenebenen definieren. Dies hat insbesondere den Vorteil, dass dann, wenn das Gelenkelement entsprechend geneigte Gelenkflächenbereichsebenen aufweist, diese flächig an den Gelenkelementanlageflächenabschnitten anliegen und so minimale Flächenpressungen erreicht werden können.

Insbesondere ist es vorteilhaft, wenn die Gelenkelementanlageflächenebenen parallel zu den Gelenkflächenbereichsebenen verlaufen. So kann auf einfache Weise das Gelenkelement flächig am Rückstellglied anliegen.

Vorzugsweise fallen die Gelenkelementanlageflächenebenen in der Grundstellung mit den Gelenkflächenbereichsebenen zusammen. Mit anderen Worten bedeutet dies, dass in der Grundstellung die fünfte Gelenkfläche flächig oder im Wesentlichen flächig am mindestens einen Rückstellglied anliegen kann.

Günstig ist es, wenn das Gelenkelement aus Polyetheretherketon (PEEK), Kobalt-Chrom, Implantat-Stahl, Titan, Keramik, Polyethylen (PE), Silikon, Hydrogel, Polytetrafluorethylen (PTFE) oder Polyethylenterephthalat (PET) hergestellt ist. Insbesondere die elastischen Materialien der genannten Werkstoffe eignen sich hervorragend, um dem Gelenkelement zusätzlich Dämpfungseigenschaften zuzuordnen. Beispielsweise kann so das Gelenkelement selbst auch einen Teil der Rückstelleinrichtung bilden, beispielsweise einen Teil des Rückstellglieds selbst.

Vorteilhafterweise sind das erste und/oder das zweite Wirbelkörperanlageelement aus Polyetheretherketon (PEEK), Kobalt-Chrom, Implantat-Stahl, Titan und/oder einer Keramik hergestellt. Die Wahl der genannten Werkstoffe ermöglicht es, Gleitpaarungen mit dem Gelenkelement aus denselben Werkstoffen auszubilden.

Vorzugsweise sind die erste und/oder die zweite und/oder die dritte und/oder die vierte und/oder die fünfte Gelenkfläche mit einer Keramik-Beschichtung versehen. Eine solche Beschichtung, welche reibungsmindernde Eigenschaften aufweist, wird vorzugsweise auf Gelenkelementen und/oder ersten und/oder zweiten Wirbelkörperanlageelementen aufgebracht, wenn diese selbst nicht aus einer Keramik hergestellt sind, um definierte keramische Gleitpaarungen hoher Abriebfestigkeit auszubilden.

Besonders einfach wird der Aufbau der Rückstelleinrichtung, wenn das Rückstellglied aus mindestens einem elastisch federnd ausgebildeten Federelement ausgebildet ist. Es können insbesondere mehrere Federelemente vorgesehen sein, welche in unterschiedlichen Raumrichtungen wirken, die insbesondere auch linear unabhängige Richtungen definieren können.

Vorteilhaft kann es ferner auch sein, wenn das mindestens eine elastisch ausgebildete Federelement aus einem Elastromer hergestellt oder in Form einer Schraubenfeder, eines Hydrogelelements oder eines Hydraulikzylinders ausgebildet ist. Je nach gewünschter Form des Rückstellglieds beziehungsweise auch des Gelenkelements und des zweiten Wirbelkörperanlageelements eignen sich die genannten Rückstellgliedvarianten besonders gut zur Ausbildung eines kompakten Bandscheibenprothesensystems.

Eine besonders kompakte Ausgestaltung des Bandscheibenprothesensystems kann insbesondere dadurch erreicht werden, dass das Gelenkelement das Rückstellglied bildet oder umfasst. Insbesondere kann das Gelenkelement einstückig ausgebildet oder zweiteilig ausgebildet sein, wobei der eine Teil des Gelenkelements die zweite Gelenkfläche, der andere Teil des Gelenkelements das Rückstellglied definieren kann.

Günstig ist es, wenn mindestens eines der Wirbelkörperanlageelemente mindestens ein Verankerungselement zum Verankern des mindestens einen Wirbelkörperanlageelements an einem Wirbelkörper aufweist. Auf diese Weise kann verhindert werden, dass sich die Prothesenkomponenten relativ zu den Wirbelkörpern bewegen können. Eine Relativbewegung zwischen den beiden Wirbelkörpern kann so in definierter Weise vom ersten Wirbelkörperanlageelement auf das Gelenkelement und von diesem auf das zweite Wirbelkörperanlageelement übertragen werden oder umgekehrt.

Besonders einfach im Aufbau wird das Bandscheibenprothesensystem, wenn dass mindestens eine Verankerungselement in Form eines Vorsprungs ausgebildet ist.

Eine besonders sichere Verankerung der Wirbelkörperanlageelemente an den Wirbelkörpern kann auf einfache Weise dadurch erreicht werden, dass der Vorsprung keil- oder spitzenförmig ausgebildet ist. Insbesondere kann er auch kegel- oder pyramidenförmig ausgebildet sein.

Die Stabilität der Wirbelkörperanlageelemente kann auf einfache Weise dadurch erhöht, dass das mindestens eine Verankerungselement einstückig mit dem mindestens einen Wirbelkörperanlageelement ausgebildet ist.

Ferner ist es denkbar, insbesondere um eine Implantation des Bandscheibenprothesensystems zu vereinfachen, dass das mindestens eine Verankerungselement in Form eines separaten, mit dem mindestens einen Wirbelkörperanlageelement verbindbaren Bauteils ausgebildet ist.

Besonders einfach lassen sich die Wirbelkörperanlageelemente an den Wirbelkörpern verankern, wenn das mindestens eine Verankerungselement in Form einer Schraube oder einer Klammer ausgebildet ist. Vorzugsweise sind entsprechende Ausnehmungen oder Aufnahmen für das mindestens eine Verankerungselement an den Wirbelkörperanlageelementen vorgesehen.

Vorteilhafterweise umfasst das Bandscheibenprothesensystem eine Kugelgelenkanschlageinrichtung zum Begrenzen einer Relativbewegung der Gelenkelemente und der zugeordneten ersten Wirbelkörperanlageelemente relativ zueinander.

Besonders einfach ausbilden lässt sich die Kugelgelenkanschlageinrichtung, wenn sie einen am Gelenkelement und/oder am ersten Wirbelkörperanlageelement ausgebildeten Vorsprung umfasst. Der Vorsprung kann jeweils einen Anschlag für das andere Teil der Prothesenkomponente bilden.

Um insbesondere eine laterale Biegebewegung des Bandscheibenprothesensystems in definierter Weise zu begrenzen, ist es vorteilhaft, wenn der Vorsprung jeweils lateralseitig an der jeweiligen Prothesenkomponente angeordnet ist. Insbesondere kann so ein einem natürlichen Bewegungsbereich nachgebildeter Bewegungsbereich des Bandscheibenprothesensystems definiert werden.

Gemäß einer weiteren bevorzugten Ausführungsform der Erfindung kann ferner vorgesehen sein, dass die Kugelgelenkanschlageinrichtung einstückig mit den jeweiligen Gelenkelementen und/oder den jeweiligen ersten Wirbelkörperanlageelementen ausgebildet ist. Diese Ausgestaltung wirkt sich günstig auf eine Stabilität der Bauteile der Prothesenkomponenten aus. Ferner können so auch besonders kompakte Bauformen der Prothesenkomponenten erreicht werden.

Die nachfolgende Beschreibung bevorzugter Ausführungsformen der Erfindung dient im Zusammenhang mit den Zeichnungen der näheren Erläuterung. Es zeigen:
- Figur 1:: eine perspektivische Gesamtansicht eines Wirbelsäulensegments mit einem zwei Prothesenkomponenten umfassenden Bandscheibenprothesensystem;
- Figur 2:: eine perspektivische Explosionsdarstellung einer rechtseitigen Prothesenkomponente des Bandscheibenprothesensystems;
- Figur 3:: eine weitere perspektivische Explosionsdarstellung der Prothesenkomponente des Bandscheibenprothesensystems aus Figur 2;
- Figur 4:: eine Querschnittsansicht durch das Bandscheibenprothesensystem in der Grundstellung in anteriorer Richtung;
- Figur 5A:: eine Schnittansicht analog Figur 4 bei einer rechtseitigen Biegung des Wirbelsäulensegments;
- Figur 5B:: eine schematische Prinzipskizze des Aufbaus des Bandscheibenprothesensystems in einer Seiten- oder Schnittansicht analog Figur 4;
- Figur 6:: eine Schnittansicht längs Linie 6-6 in Figur 4;
- Figur 7A:: eine Schnittansicht analog Figur 6 in einer Extensionsstellung;
- Figur 7B:: eine schematische Prinzipskizze des Bandscheibenprothesensystems entsprechend der Schnittansicht in Figur 6;
- Figur 8A:: eine Draufsicht auf das Bandscheibenprothesensystem von oben bei einer axialen Rotation um eine Längsachse der Wirbelsäule;
- Figur 8B:: eine schematische Prinzipskizze zur in Figur 8A dargestellten Auslenkung des Bandscheibenprothesensystems;
- Figur 9A:: eine Prinzipdarstellung einer weiteren Ausführungsform eines Bandscheibenprothesensystems in einer Ansicht von vorn (in anterior-posteriorer Richtung);
- Figur 9B:: eine schematische Seitenansicht des Bandscheibenprothesensystems aus Figur 9A von der Seite von lateral;

- Figur 10A:: eine schematische Prinzipskizze analog Figur 9A einer weiteren Variante eines Bandscheibenprothesensystems;
- Figur 10B:: eine Prinzipskizze analog Figur 9B des Bandscheibenprothesensystems aus Figur 10A;
- Figur 11A:: eine Prinzipskizze analog Figur 9A einer weiteren Ausführungsform eines Bandscheibenprothesensystems;
- Figur 11B:: eine Prinzipskizze analog Figur 9B des in Figur 11A dargestellten Bandscheibenprothesensystems;
- Figur 12A:: eine Prinzipskizze analog Figur 9A eines weiteren Ausführungsbeispiels eines Bandscheibenprothesensystems; und
- Figur 12B:: eine Prinzipskizze analog Figur 9B des in Figur 12A dargestellten Bandscheibenprothesensystems.

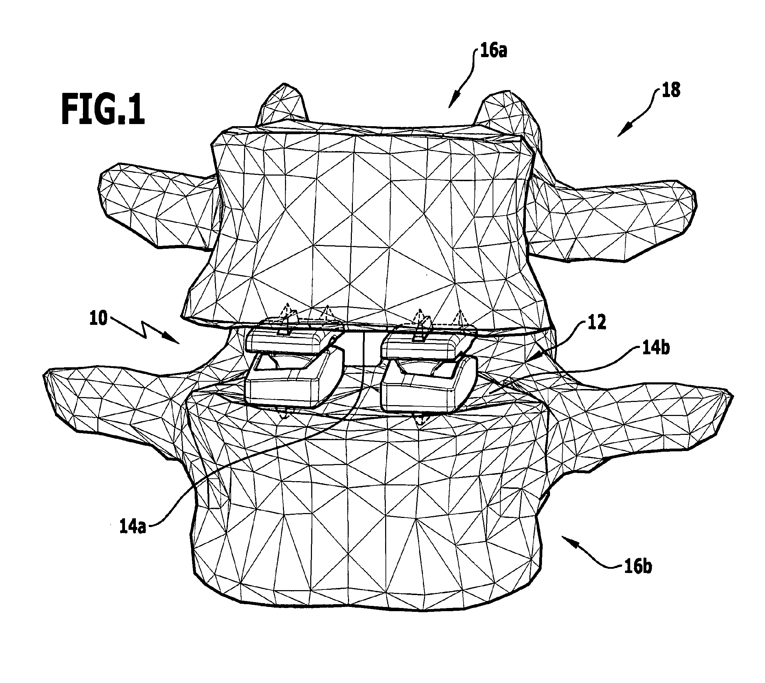

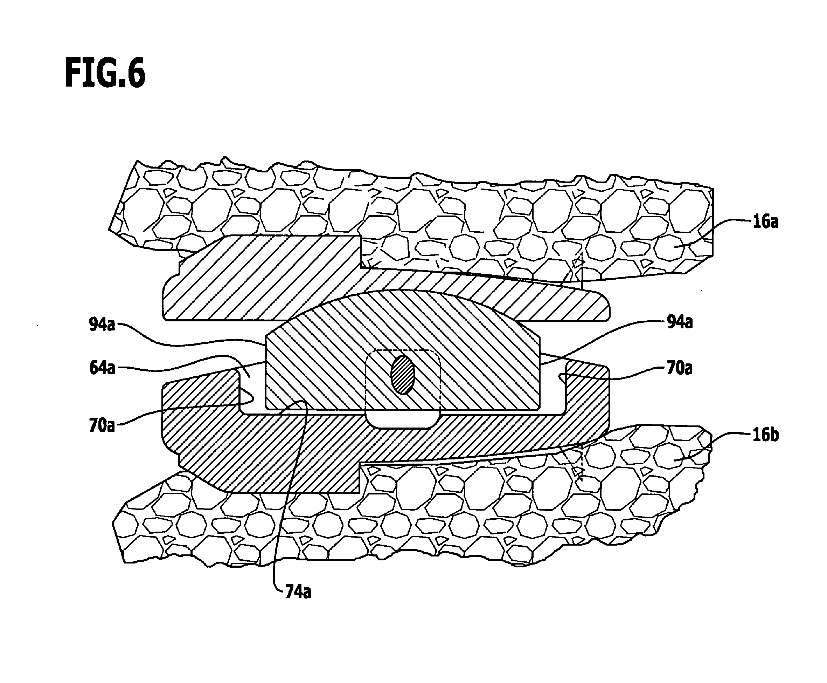

In den Figuren 1 bis 8 ist ein insgesamt mit dem Bezugszeichen 10 versehenes Bandscheibenprothesensystem zum Einsetzen in einen Bandscheibenraum 12 nach einer Resektion oder Teilresektion einer natürlichen Bandscheibe dargestellt. Der Bandscheibenraum 12 wird seitlich begrenzt durch Gelenkflächen 14a und 14b benachbarter Wirbelkörper 16a und 16b einer Wirbelsäule 18.

Das Bandscheibenprothesensystem 10 umfasst zwei Prothesenkomponenten 20a und 20b, welche spiegelsymmetrisch zu einer Medianebene 22 ausgebildet sind. Die Prothesenkomponente 20a wird vorzugsweise in der rechten Körperhälfte des Patienten, die Prothesenkomponente 20b vorzugsweise in der linken Körperhälfte des Patienten implantiert.

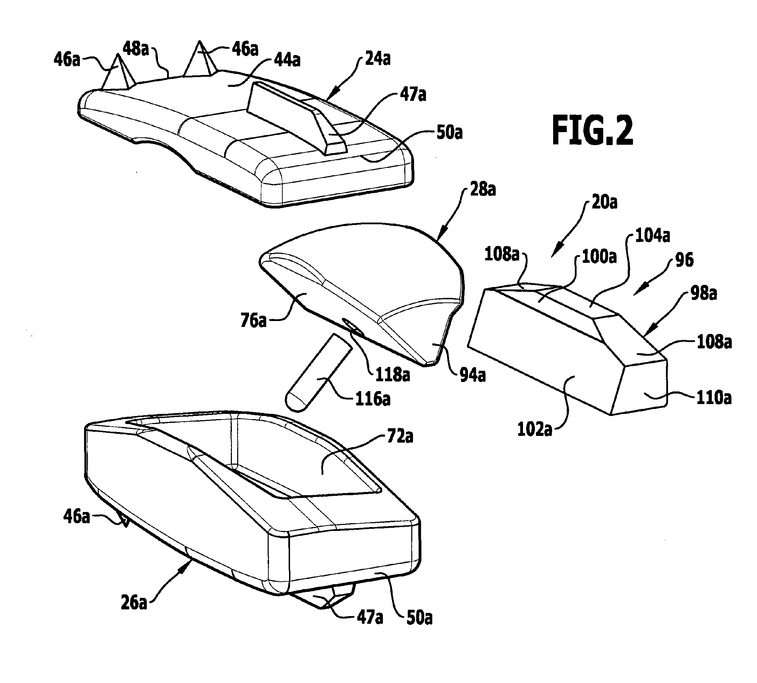

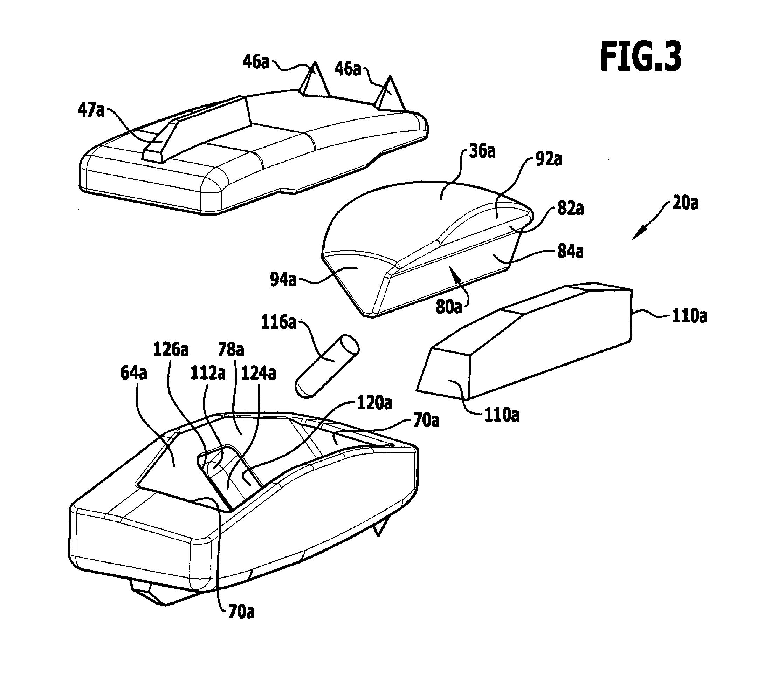

Der Aufbau der Prothesenkomponenten 20 und 22 wird nachfolgend insbesondere mit Verweis auf die Figuren 2 und 3 näher erläutert. Identische beziehungsweise spiegelsymmetrisch ausgebildete Elemente der Prothesenkomponenten 20a und 20b sind der Übersichtlichkeit wegen mit denselben Bezugszeichen versehen und unterscheiden sich lediglich im Endbuchstaben a beziehungsweise b. Aufgrund der spiegelsymmetrischen Ausbildung der Prothesenkomponenten 20a und 20b beschränkt sich die nachfolgende detaillierte Beschreibung auf die Ausgestaltung der rechtsseitigen Prothesenkomponente 20a.

Die Prothesenkomponente 20a umfasst ein erstes Wirbelkörperanlageelement 24a, ein zweites Wirbelkörperanlageelement 26a sowie ein an beiden Wirbelkörperanlageelementen 24a und 26a beweglich gelagertes Gelenkelement 28a. Des Weiteren umfasst die Prothesenkomponente 20a eine Bewegungsbegrenzungseinrichtung 30 zum Begrenzen einer Relativbewegung des Gelenkelements 28a und des zweiten Wirbelkörperanlageelements 26a. Ferner umfasst die Prothesenkomponente 20a ein zwischen den ersten und zweiten Wirbelkörperanlageelementen 24a und 26a ausgebildetes Kugelgelenk 32a. Das Kugelgelenk 32a wird definiert durch das erste Wirbelkörperanlageelement 24a und das zugeordnete Gelenkelement 28a. Zur Ausbildung des Kugelgelenks 32a definiert das erste Wirbelkörperanlageelement eine erste Gelenkfläche 34a und das Gelenkelement 28a eine zweite Gelenkfläche 36a, die korrespondierend zur ersten Gelenkfläche 34a ausgebildet ist. Die zweite Gelenkfläche 36a bildet einen Teil einer Kugeloberfläche und die erste Gelenkfläche 34a einen Teil einer Hohlkugeloberfläche. Radien der Kugeloberfläche und der Hohlkugeloberfläche sind identisch und definieren einen Mittelpunkt 38a des Kugelgelenks 32a, welcher nach einer Implantation im Wesentlichen im Innern des Wirbelkörpers 16b liegt. Die erste Gelenkfläche 34a weist in Richtung auf die Gelenkfläche 14b hin, die zweite Gelenkfläche 36a in Richtung auf die Gelenkfläche 14a.

Des Weiteren ist die Prothesenkomponente 20a mit einer Kugelgelenkanschlageinrichtung 40a ausgestattet zum Begrenzen einer Relativbewegung des Gelenkelements 28a und des zugeordneten ersten Wirbelkörperanlageelements 24a relativ zueinander. Die Kugelgelenkanschlageinrichtung 40a umfasst einen am ersten Wirbelkörperanlageelement 24a lateralseitig ausgebildeten Vorsprung 42a, welcher im Wesentlichen in Form eines in Richtung auf das zweite Wirbelkörperanlageelement 26a hin weisenden Randes oder Flansches ausgebildet ist. Der Vorsprung 42a begrenzt eine Bewegung des Gelenkelements 28a in lateraler Richtung beziehungsweise eine Bewegung des ersten Wirbelkörperanlageelements 24a in Richtung auf die Prothesenkomponente 20a hin. Der Vorsprung 42a könnte optional auch medialseitig am ersten Wirbelkörperanlageelement angeordnet sein. Optional kann die Kugelgelenkanschlageinrichtung 40a auch einen nicht dargestellten Vorsprung am Gelenkelement 28a umfassen, der in ähnlicher beziehungsweise analoger Weise mit dem ersten Wirbelkörperanlageelement 24a zusammenwirkt. Er kann lateral- oder medialseitig am Gelenkelement 28a angeordnet sein. Vorzugsweise ist der Vorsprung 42a, wie bei dem in den Figuren dargestellten Ausführungsbeispiel des Bandscheibenprothesensystems 10, einstückig mit dem ersten Wirbelkörperanlageelement 24a ausgebildet.

Das erste Wirbelkörperanlageelement 24a ist insgesamt im Wesentlichen plattenförmig ausgebildet und weist eine schwach konvex in Richtung auf die Gelenkfläche 14a hin weisende und an diese anlegbare Gelenkanlagefläche 44a auf. Um eine sichere Verankerung des ersten Wirbelkörperanlageelements 24a am Wirbelkörper 16a sicherstellen zu können, sind von der Gelenkanlagefläche 44a in Richtung auf die Gelenkfläche 14a im Wesentlichen senkrecht abstehende Verankerungselemente 46a und 47a vorgesehen. Zwei pyramidenförmige Verankerungselemente 46a sind im Bereich einer posterioren Hinterkante 48a beabstandet voneinander angeordnet, das Verankerungselement 47a ist in Form einer Verankerungsrippe oder eines keilförmigen Vorsprungs ausgebildet, welches sich ausgehend von einer anterioren Vorderkante 50a etwa über die Hälfte der Länge des ersten Wirbelkörperanlageelement 24a in anterior-posteriorer Richtung erstreckt. Insgesamt ist das Verankerungselement 47a finnenartig ausgebildet.

In analoger Weise sind auch am zweiten Wirbelkörperanlageelement 26a Verankerungselemente 46a und 47a ausgebildet, nämlich zwei Verankerungselemente 47a im Bereich einer Hinterkante 48a des flachen, quaderförmigen zweiten Wirbelkörperanlageelements 26a in Form pyramidenförmiger und senkrecht von einer Gelenkanlagefläche 45a abstehender Vorsprünge. Die Gelenkanlagefläche 45a ist analog zur Gelenkanlagefläche 44a schwach konvex in Richtung auf den Wirbelkörper 16b hin gekrümmt. Ausgehend von einer Vorderkante 50a des zweiten Wirbelkörperanlageelements 26a erstreckt sich auf der Gelenkanlagefläche 45a über etwa die Hälfte einer Erstreckung des zweiten Wirbelkörperanlageelements 26a in anterior-posteriorer Richtung ein rippenartiger oder finnenartiger Vorsprung, welcher das Verankerungselement 47a ausbildet. Alle Verankerungselemente 47a sind schwach keilförmig und verjüngen sich im Querschnitt in einer Richtung vom jeweiligen Wirbelkörperanlageelement 24a beziehungsweise 26a weg. Optional können auch nicht dargestellte Verankerungselemente in Form separater, mit den Wirbelkörperanlageelementen 24a beziehungsweise 26a verbindbarer Bauteile vorgesehen sein. Denkbar sind insbesondere Schrauben oder Klammern, welche vorzugsweise in entsprechende mit Ausnehmungen oder Aufnahmen an den Wirbelkörperanlageelementen 24a beziehungsweise 26a ein- oder durchgeführt werden können.

Das Bandscheibenprothesensystem 10 umfasst ferner eine Führungseinrichtung 52 zum Definieren eines übergeordneten, durch die Prothesenkomponenten 20a und 20b gemeinsam definierten Drehgelenks 54 zum gleichzeitigen Verdrehen der beiden ersten Wirbelkörperanlageelemente 24a und 24b relativ zu den beiden zweiten Wirbelkörperanlageelementen 26a und 26b um ein gemeinsames Drehzentrum 56. Das Drehzentrum 56 liegt in einem Raumbereich 58 zwischen den Mittelpunkten 38a und 38b der Kugelgelenke 32a und 32b. Der Raumbereich 58 wird zumindest in einer Grundstellung des Bandscheibenprothesensystems 10, wie es in den Figuren 1, 4 und 6 dargestellt ist, von zwei senkrecht zu einer Verbindungslinie 60 der beiden Mittelpunkte der beiden Kugelgelenke 32a und 32b verlaufenden Ebenen 62a und 62b begrenzt.

Die Führungseinrichtung 52 umfasst an jeder Prothesenkomponente 20a und 20b jeweils eine Führungsfläche 64a und 64b zum Führen einer Bewegung der beiden Kugelgelenke 32a und 32b relativ zum zweiten Wirbelkörperanlageelement 26a beziehungsweise 26b. Die Führungsflächen 64a und 64b sind ausgebildet zum Führen einer Bewegung der jeweiligen Gelenkelemente 28a und 28b relativ zum zweiten Wirbelkörperanlageelement 26a und 26b. Sie sind bei dem in den Figuren dargestellten Ausführungsbeispiel des Bandscheibenprothesensystems 10 am zweiten Wirbelkörperanlageelement 26a beziehungsweise 26b ausgebildet, können jedoch auch am Gelenkelement 28a beziehungsweise 28b ausgebildet sein. Die Führungsflächen 64a und 64b sind relativ zu den Ebenen 62a und 62b um einen Neigungswinkel 66a beziehungsweise 66b geneigt, und zwar jeweils in lateraler Richtung und auf das erste Wirbelkörperanlageelement 24a beziehungsweise 24b hin weisend. Der Neigungswinkel 66a, 66b beträgt bei dem in den Figuren dargestellten Bandscheibenprothesensystem 10 45°, kann jedoch auch in einem Bereich von 20° bis 70° liegen.

Die Führungsfläche 64a, 64b bildet eine seitliche Begrenzungsfläche einer Aufnahme 68a des zweiten Wirbelkörperanlageelements 26a beziehungsweise 26b für das jeweilige Gelenkelement 28a und 28b. Die Führungsfläche 64a, 64b wird anteriorseitig sowie posteriorseitg durch innere, einander gegenüberliegende Stirnflächen 70a, 70b begrenzt, welche im Wesentlichen parallel zueinander und senkrecht zu den Ebenen 62a und 62b verlaufen. Eine laterale innere Seitenfläche 72a, 72b ist senkrecht oder im Wesentlichen senkrecht zu den Stirnflächen 70a, 72b orientiert und verläuft im Wesentlichen parallel oder parallel zu den Ebenen 62a und 62b. Die Aufnahme 68a, 68b umfasst ferner eine im Wesentlichen langgestreckt rechteckige Bodenfläche 74a, 74b, welche gemeinsame Kanten mit der Führungsfläche 64a beziehungsweise 64b sowie mit den Stirnflächen 70a beziehungsweise 70b sowie den Seitenflächen 72a beziehungsweise 72b aufweist.

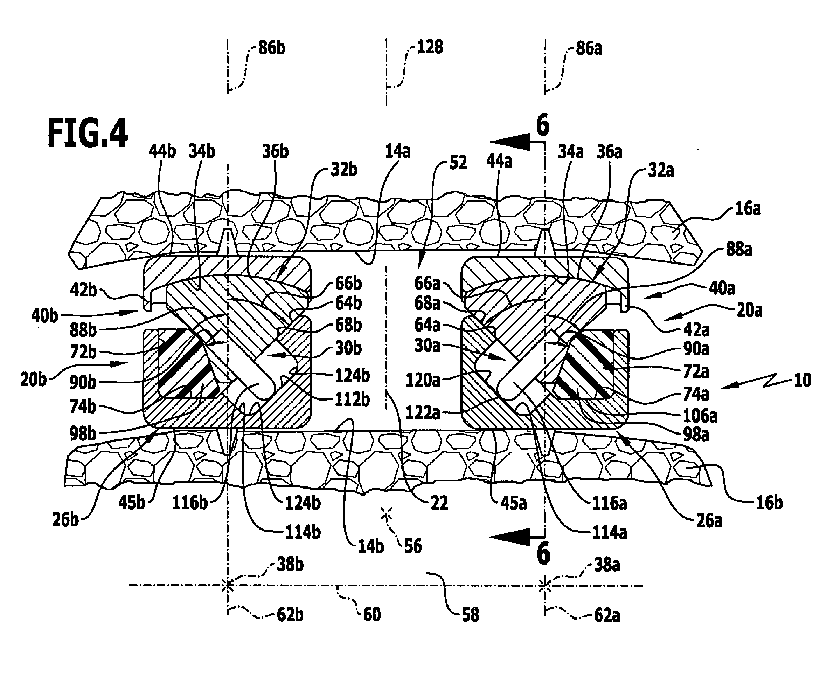

Das Gelenkelement 28a definiert eine vierte ebene Gelenkfläche 76a und das zweite Wirbelkörperanlageelement 26a eine dritte Gelenkfläche 78a, welche die Führungsfläche 64a bildet. Das Gelenkelement 28a ist im Wesentlichen im Querschnitt, wie beispielsweise in Figur 4 dargestellt, keilförmig beziehungsweise prismenförmig ausgebildet. Zwei Seitenflächen des Gelenkelements 28a werden gebildet durch die bereits beschriebene zweite Gelenkfläche 36a sowie die vierte Gelenkfläche 76a. Eine weitere, nämlich die dritte Keilfläche des Gelenkelements 28a bildet eine fünfte Gelenkfläche 80a, welche zwei relativ zueinander geneigte Gelenkflächenbereiche 82a und 84a aufweist. Die vierte Gelenkfläche 76a der Prothesenkomponente 20a ist in medialer Richtung geneigt, die fünfte Gelenkfläche 80a in lateraler Richtung. Der Gelenkflächenbereich 82a ist relativ zur Ebene 62a und damit zu einer vom zweiten Wirbelkörperanlageelement 26a definierten Längsachse 86a stärker geneigt als eine durch die Führungsfläche 64a definierte Gelenkebene. Der Gelenkflächenbereich 84a ist bezogen auf die Längsachse 86a weniger stark geneigt als die Gelenkebene. Ein Neigungswinkel 88a des Gelenkflächenbereichs 82a zur Ebene 62a beträgt etwa 50°, kann aber auch in einem Bereich von 40° bis 60° liegen. Ein Neigungswinkel 90a des Gelenkflächenbereichs 84a bezogen auf die Ebene 62a beträgt circa 20°, kann jedoch aber auch in einem Bereich von 10° bis 30° liegen.

Schnittlinien von durch die Gelenkflächenbereiche 82a und 84a definierte Gelenkflächenbereichsebenen verlaufen in anterior-posteriorer Richtung, ebenso wie eine Schnittlinie, die durch die vom Gelenkflächenbereich 84a definierte Gelenkflächenbereichsebene und die von der vierten Gelenkfläche 76a definierten Ebene. Ferner weist das Gelenkelement 28a eine Anschlagfläche 92a auf, die im Wesentlichen parallel zur Ebene 62a verläuft und mit dem Gelenkflächenbereich 62a eine gemeinsame Schnittlinie, welche in anterior-posteriorer Richtung verläuft, definiert. Die Anschlagfläche 92a wirkt mit dem Vorsprung 42a zusammen und kann mit dieser bei einer entsprechenden Relativbewegung des Gelenkelements 28a und des ersten Wirbelkörperanlageelements 24a in Kontakt kommen.

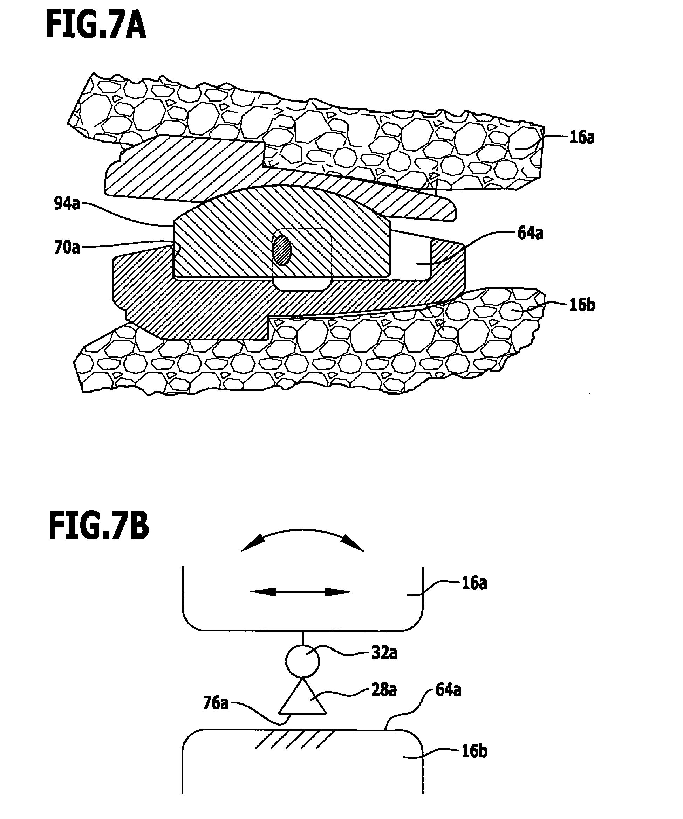

Das Gelenkelement 28a weist ferner im Wesentlichen dreieckige Stirnflächen 94a auf, welche in anteriorer und posteriorer Richtung weisen und im Wesentlichen parallel zu den Stirnflächen 76a verlaufen. Ein Abstand zwischen den Stirnflächen 94a ist etwas kürzer als ein Abstand zwischen den Stirnflächen 70a, wodurch eine Translationsbewegung des Gelenkelements 28a in anteriorer beziehungsweise posteriorer Richtung in der Aufnahme 68a möglich ist, bis die Stirnflächen 94a an den jeweiligen Stirnflächen 70a anschlagen, wie dies beispielsweise in Figur 7A dargestellt ist.

Das Bandscheibenprothesensystem 10 umfasst ferner einer Rückstelleinrichtung 96 zum Rückführen der beiden Kugelgelenke 32a und 32b der Prothesenkomponenten 20a und 20b von einer aus einer Grundstellung ausgelenkten Stellung zurück in die Grundstellung. Die Grundstellung des Bandscheibenprothesensystems 10 ist beispielsweise in Figur 4 dargestellt. Die beiden Prothesenkomponenten 20a und 20b sind in der Grundstellung spiegelsymmetrisch zu der eine Symmetrieebene definierenden Medialebene 22 ausgebildet. Die Rückstelleinrichtung 96 umfasst zwei Rückstellglieder 98a und 98b, welche im Wesentlichen quaderförmig ausgebildet und in die Aufnahmen 68a, 68b derart eingesetzt sind, dass sie sie teilweise ausfüllen. Insbesondere liegen Seitenflächen des Rückstellglieds 98a flächig an den Stirnflächen 70a, der Seitenfläche 72a sowie der Bodenfläche 74a an. Ferner ist das Rückstellglied 98a derart ausgebildet, dass es flächig an der fünften Gelenkfläche 80a anliegt, und zwar mit entsprechend geneigten Rückstellgliedflächenbereichen 100a und 102a, wobei in der Grundstellung der Rückstellgliedflächenbereich 100a am Gelenkflächenbereich 82a und der Rückstellgliedflächenbereich 102a am Gelenkflächenbereich 84a anliegt. Eine Oberseite 104a des Rückstellglieds 98a verläuft parallel zu einer an der Bodenfläche 74a anliegenden Unterseite 106a. Des Weiteren ist das Rückstellglied 98a anterior- und posteriorseitig mit bezogen auf die Oberseite 104 in etwa um 10° geneigten Abflachungen 108a in Richtung auf anteriore und posteriore Stirnflächen 110a des Rückstellglieds 98a hin versehen, welche insbesondere bei Flexion/Extension zusätzliche Anschlagflächen für das Gelenkelement 28a bilden. Die Stirnflächen 110a liegen flächig an den Stirnflächen 70a an. Vorzugsweise ist das Rückstellglied 98a unlösbar mit dem zweiten Wirbelkörperanlageelement verbunden.

Das Rückstellglied 98a ist aus mindestens einem elastisch federnd ausgebildeten Federelement ausgebildet, welches vorzugsweise aus einem Elastomer hergestellt ist. Es kann sich aber auch um eine oder mehrere Schraubenfedern oder um ein Hydrogelelement mit der oben beschriebenen Form handeln. Denkbar sind auch entsprechende, nicht dargestellte Hydraulikzylinder. Die Rückstelleinrichtung 96 übt gleichzeitig auch die Funktion einer Dämpfungseinrichtung aus. Wird das Gelenkelement 28a gegen das Rückstellglied 98a gedrückt, wird das Rückstellglied 98 elastisch verformt und in einer Richtung in die Aufnahme 68a zurückgedrängt, in welche im Wesentlichen die einwirkende Kraft weist.

Die Bewegungsbegrenzungseinrichtung 30a umfasst erste und zweite Anschlagglieder 112a und 114a. Das erste Anschlagglied 112a ist in Form eines zylindrischen Anschlagstifts 116a ausgebildet, welcher in ein senkrecht zur vierten Gelenkfläche 76a am Gelenkelement 28a ausgebildetes Sackloch 118a eingesetzt ist und so weit über die vierte Gelenkfläche 76a übersteht wie eine das erste Anschlagglied 112a definierende quaderförmige Ausnehmung 120a tief ist. Ein freies Ende 122a des Anschlagstifts 116a ist halbkugelig geformt. Innenkanten 124a der Ausnehmung 120a sind entsprechend dem Radius des freien Endes 122 verrundet. Die Ausnehmung 120a definiert im Querschnitt einen im Wesentlichen rechteckigen Flächenbereich 126a in der Führungsfläche 64a. Eine Länge und eine Breite des Flächenbereichs 126a sind größer als ein Durchmesser des Anschlagstifts 116a, so dass das Gelenkelement 128a auf der Führungsfläche 64a sowohl translatorisch als auch rotatorisch, insbesondere um eine durch den Anschlagstift 116a definierte Längsachse, bewegt werden kann, soweit dies die Bewegungsbegrenzungseinrichtung 30 zulässt. Eine Bewegungsbegrenzung erfolgt durch Anschlagen des Anschlagstifts 116a an Seitenflächen der Ausnehmung 120a. Auf diese Weise kann eine Bewegung des Gelenkelements 28a sowohl in posteriorer als auch in anteriorer Richtung sowie in Richtung auf das Rückstellglied 98a hin als auch von diesem weg in definierter Weise begrenzt werden. Ein Querschnitt des ersten Anschlagglieds 112a ist größer als ein Querschnitt des zweiten Anschlagglieds 114a. Das zweite Anschlagglied 114a ist somit innerhalb des Flächenbereichs 126a frei beweglich.

Die Funktionsweise des Bandscheibenprothesensystems 10 wird nachfolgend im Zusammenhang mit den Figuren 1 bis 8B näher erläutert.

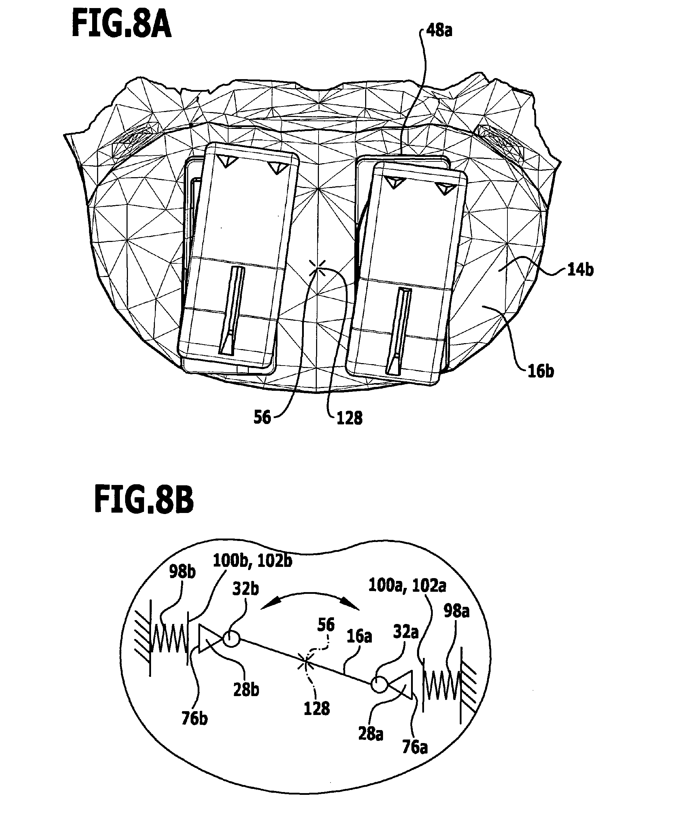

Die beiden Prothesenkomponenten 20a und 20b werden über einen bilateralen Zugang von posterior zwischen den Laminae, also durch einen interlaminaren Zugang, implantiert. Vorzugsweise werden sie parallel zueinander in anterior-posteriorer Richtung eingesetzt, wie dies in den Figuren 1 und 8A dargestellt ist. Das Bandscheibenprothesensystem 10 ist insgesamt derart ausgebildet, dass eine Position des Drehzentrums 56 im Raumbereich 58 in definierter Weise in Abhängigkeit einer Auslenkung des Bandscheibenprothesensystems aus der Grundstellung, in welcher es symmetrisch konfiguriert ist, veränderbar ist.

Die beiden Prothesenkomponenten 20a und 20b bilden zwei im weitesten Sinne unabhängig voneinander wirkende Funktionseinheiten, welche jeweils als Einheit mittels eines geeigneten Einsetzinstruments in den Bandscheibenraum 12 eingesetzt werden können, wobei mit einer Einheit der Bandscheibenraum 12 distrahierbar ist. Die beiden Kugelgelenke 32a und 32b weisen keinen gemeinsamen Drehpunkt auf. Die ersten und zweiten Gelenkflächen 34a, 34b sowie 36a und 36b bilden aufgrund des einander entsprechenden Krümmungsradius flächige Gleitpaarungen. Eine weitere Gleitpaarung wird ausgebildet zwischen den dritten und vierten Gelenkflächen 78a, 78b und 76a, 76b. Die Führungsfläche 64a, 64b ermöglicht es aufgrund des Neigungswinkels 66a, 66b dem Gelenkelement 28a, 28b zum einen, sich bei einem axialen Moment bezogen auf eine von dem durch die Wirbelkörper 16a und 16b sowie das Bandscheibenprothesensystem 10 definierten Wirbelsäulensegment definierte Längsachse 128 in anteriorer beziehungsweise posteriorer Richtung zu bewegen. In Kombination mit einer Rotation in den Kugelgelenken 32a und 32b kann somit eine überlagerte axiale Rotationsbewegung des Bandscheibenprothesensystems 10 um die senkrecht zur Verbindungslinie 60 verlaufende Längsachse 128 erreicht werden. Eine entsprechende Auslenkung der Kugelgelenke 32a und 32b ist in der Figur 8A dargestellten Draufsicht zu erkennen. In Figur 8B ist die Prinzipskizze der axialen Rotation schematisch dargestellt.

Auch eine Flexion beziehungsweise Extension kann mittels des Bandscheibenprothesensystems 10 erreicht werden, und zwar durch Rotation um die Verbindungslinie 60 der Mittelpunkte 38 und 38b. Eine Begrenzung der axialen Rotationsbewegung erfolgt zum einen über die aneinander anschlagenden Stirnflächen 70a, 70b und 94a, 94b. Zum anderen bildet auch die Bewegungsbegrenzungseinrichtung 30 mit den Anschlaggliedern 112a, 112b und 114a, 114b eine Begrenzung der Bewegung der Gelenkelemente 28a, 28b in anteriorer und posteriorer Richtung.

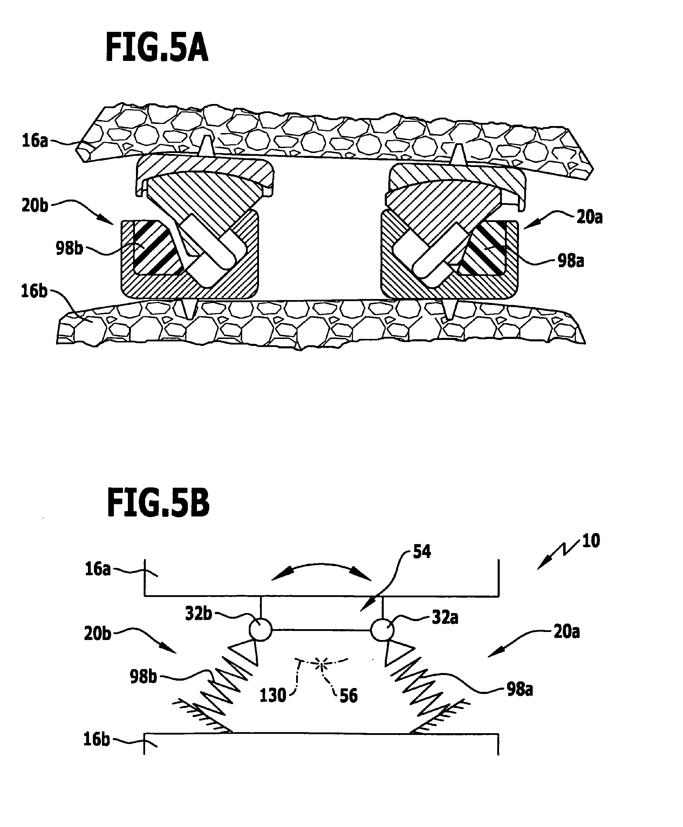

Das Bandscheibenprothesensystem 10 ermöglicht ferner eine laterale Rotationsbewegung um das Drehzentrum 56, also quer zur anterior-posterioren Richtung. Durch entsprechendes Einleiten eines Moments wird das Gelenkelement 28a in Richtung auf das Rückstellglied 98a hin gedrückt und somit das erste Wirbelkörperanlageelement 24a nach lateral verschoben und gleichzeitig durch die Führungsfläche 64a in Richtung auf das zweite Wirbelkörperanlageelement 26a hin geführt. Da eine Zwangsbedingung des Bandscheibenprothesensystems 10 ist, dass die ersten und zweiten Gelenkflächen 34a, 34b und 36a, 36b unabhängig von einer Auslenkung des Bandscheibenprothesensystems 10 aus der Grundstellung flächig aneinander anliegen, gleitet bei der beschriebenen lateralen Rotation das Gelenkelement 28b gleichzeitig an der Führungsfläche 64b auf, so dass sich das Kugelgelenk 32b in Richtung auf die Prothesenkomponente 20a hin bewegt. Das Gelenkelement 28b hebt dabei etwas vom Rückstellglied 98b ab. Eine Dämpfung erfolgt bei einer lateralen Rotation stets durch das jenige Rückstellglied, auf welchem der größere Druck lastet. Eine Begrenzung der axialen Rotation erfolgt über die Kugelgelenkanschlageinrichtung 40 beziehungsweise über die Bewegungsbegrenzungseinrichtung 30. Ein Beispiel für eine laterale Rotationsauslenkung ist in Figur 5A dargestellt, Figur 5B zeigt die schematische Prinzipskizze einer solchen lateralen Rotationsauslenkung.

Zu beachten ist ferner, dass, abhängig vom Neigungswinkel 66a, 66b, das Drehzentrum 56 längs einer Bahnkurve 130 in Abhängigkeit eines Grads der Auslenkung aus der Grundstellung wandern kann. Das Drehzentrum 56 entfernt sich somit bei dem in den Figuren dargestellten Ausführungsbeispiel von der Grundstellungsmittelebene oder Medianebene 22 mit zunehmender Auslenkung des Bandscheibenprothesensystems 10 aus der Grundstellung.

Bei einer Flexion/Extension des Wirbelsäulensegments findet eine Rotation des Bandscheibenprothesensystems 10 insgesamt um eine durch die Verbindungslinie 60 definierte Rotationsachse statt. Bei einwirkenden Momenten kann jedoch zusätzlich auch eine Translationsbewegung der Gelenkelemente 28a, 28b gemeinsam in anteriorer beziehungsweise posteriorer Richtung erfolgen. Eine Bewegung der Gelenkelemente 28a, 28b relativ zu den zweiten Wirbelkörperanlageelementen 26a, 26b wird zum einen begrenzt durch die Stirnflächen 70a, 70b und 94a, 94b sowie die Anschlagglieder 112a, 112b und 114a, 114b der Bewegungsbegrenzungseinrichtung 30. Eine schematische Prinzipskizze der Extensions-/Flexions-Bewegung ist in Figur 7B dargestellt. Eine Flexion/Extension bedingt aufgrund der Ausbildung des Bandscheibenprothesensystems 10 stets eine Rotation und eine Translationsbewegung der ersten und zweiten Wirbelkörperanlageelemente 24a, 24b und 26a, 26b relativ zueinander.

Selbstverständlich ermöglicht es das Bandscheibenprothesensystem 10 auch, Flexions-/Extensions-Bewegungen mit axialen Rotationsbewegungen zu überlagern. Auch bei einer solchen Bewegung wandert das Drehzentrum 56 längs einer definierten Bahnkurve 130 in Abhängigkeit einer Auslenkung des Bandscheibenprothesensystems 10 aus der Grundstellung. Anders als bei bekannten Bandscheibenprothesensystemen sind mit dem oben beschriebenen Bandscheibenprothesensystem 10 physiologisch gekoppelte Bewegungen möglich, insbesondere die beschriebene überlagerte Flexions-/Extensions- mit axialer Rotationsbewegung .

Aufgrund der Ausgestaltung der Prothesenkomponenten 20a, 20b ist die Funktionsweise des Bandscheibenprothesensystems 10 im Wesentlichen unabhängig von einer Position der Prothesenkomponenten 20a und 20b nach dem Implantieren. Die Bewegungsbegrenzungseinrichtung 30 sowie die Kugelgelenkanschlagseinrichtung 40 begrenzen eine Bewegung der benachbarten Wirbelkörper 16a und 16b innerhalb der physiologischen Grenzen. Die Rückstelleinrichtung 96 stellt eine gewünschte Dämpfung bei einer Bewegung der Wirbelkörper 16a, 16b relativ zueinander sicher.

Die Wirbelkörperanlageelemente 24a, 24b, 26a und 26b können insbesondere aus Polyetheretherketon (PEEK), Kobalt-Chrom, Implantat-Stahl, Titan oder einer Keramik ausgebildet sein. Insbesondere können die Gelenkflächen 34a, 34b, 36a, 36b, 78a, 78b, 76a, 76b und 80a, 80b mit einer Keramikbeschichtung zur Reibungsminderung und Erhöhung einer Abriebfestigkeit versehen sein. Die Gelenkteile 28a, 28b können insbesondere aus Polyetheretherketon (PEEK), Kobalt-Chrom, Implantat-Stahl, Titan, Keramik, Polyethylen (PE), Silikon, Hydrogel, Polytetrafluorethylen (PTFE) oder Polyethylenterephthalat (PET) hergestellt und optional bei Bedarf mit einer keramischen Beschichtung versehen sein.

Die Rückstellglieder 98a, 98b verhindern, dass sich die Gelenkteile 28a, 28b längs der Führungsfläche 64a, 64b auf Anschlag nach unten auf die Wirbelkörperanlageelemente 26a, 26b hin bewegen würden, was aufgrund einer auf eine Bandscheibe normalerweise wirkenden Kompressionskraft normalerweise erfolgen würde. Abhängig von den wirkenden Kräften dient die Rückstelleinrichtung 96 häufig weniger als Rückstelleinrichtung, sondern vielmehr als Dämpfungseinrichtung für das Bandscheibenprothesensystem 10.

Durch die keilförmige Ausgestaltung der Gelenkelemente 28a, 28b bewegt sich eine Position des Drehzentrums 56 auf einer definierten Bahnkurve 130 im Raum, was einer Nachbildung der physiologischen Bewegungsbegrenzung durch die Facettengelenke der Wirbel entspricht. Die Rückstelleinrichtung 96 ermöglicht eine kraftabhängige Kinematik des Bandscheibenprothesensystems 10, indem sie die Gelenkelemente 28a, 28b entsprechend in die Grundstellung zurückzubewegen sucht.

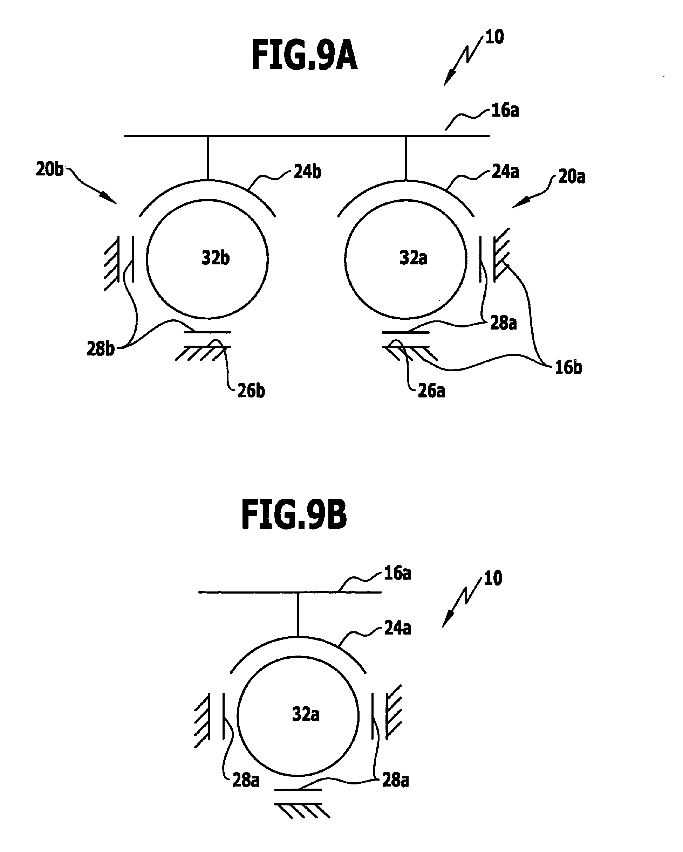

Mögliche alternative prinzipielle Konstruktionen des Bandscheibenprothesensystems sind in den Figuren 9A bis 12B schematisch dargestellt.

In den Figuren 9A und 9B ist schematisch dargestellt, dass ein Bandscheibenprothesensystem 10 mit Kugelgelenken 32a und 32b auch durch entsprechende gleitende Lagerung eines oder mehrerer Gelenkelemente 28a und 28b relativ zu den zweiten Wirbelkörperanlageelementen 26a und 26b in entsprechender Weise ausgebildet werden kann.

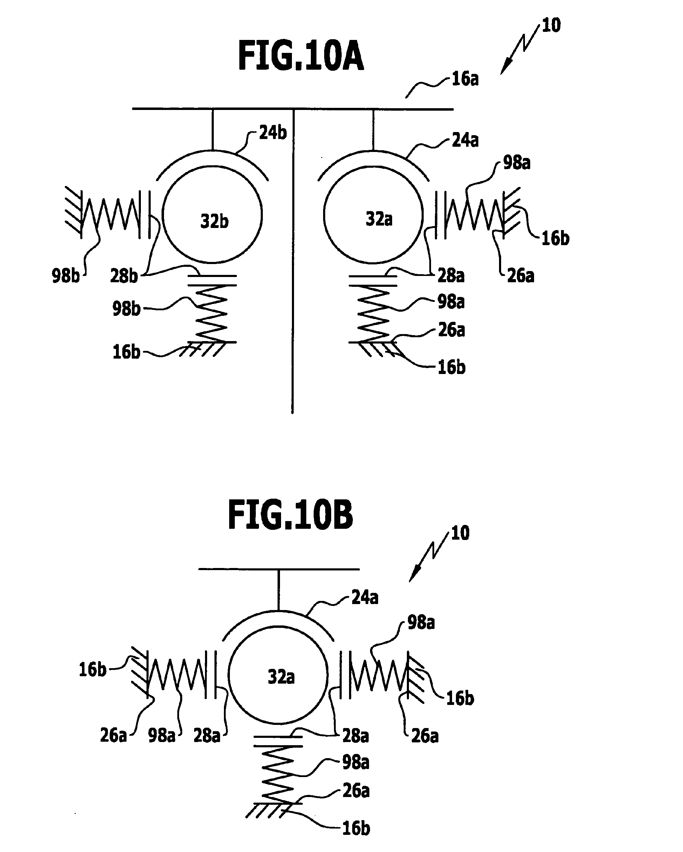

Optional können bei dem in den Figuren 10A und 10B dargestellten Bandscheibenprothesensystem 10, welches ohne eine Rückstelleinrichtung 96 ausgebildet ist, zwischen dem zweiten Wirbelkörperanlageelement 26a und 26b zusätzlich ein oder mehrere Rückstellglieder 98a, 98b geschaltet sein, die eine Bewegung der Wirbelkörper 16a und 16b relativ zueinander dämpfen.

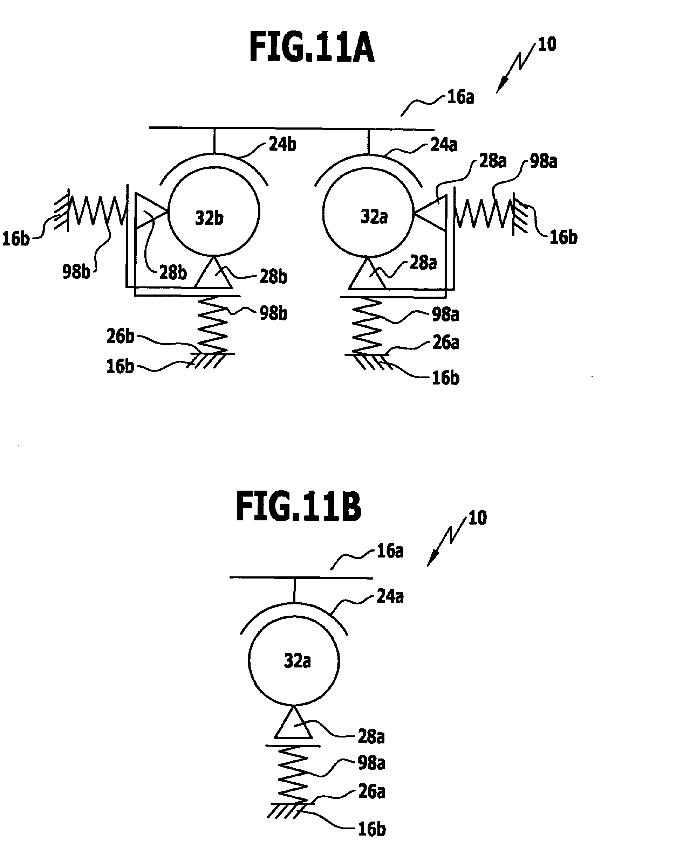

Anders als bei den in den Figuren 9A bis 10B dargestellten Bandscheibenprothesensystem 10 erfolgt eine zwangsweise Lagerung der Kugelgelenke 32a und 32b bei dem in den Figuren 11A bis 12B dargestellten Bandscheibenprothesensystemen 10 durch entsprechende Überkreuzankopplung der Gelenkelemente 28a und 28b an den Wirbelkörperanlageelementen 26a, 26b. Durch die jeweils zwei Gleitpaarungen miteinander ausbildenden Gelenkteilelemente 28a beziehungsweise 28b können so auch überlagerte Bewegungen in zwei voneinander linear unabhängigen Richtungen erreicht und mit entsprechenden Rückstellgliedern 98a, 98b gedämpft werden.

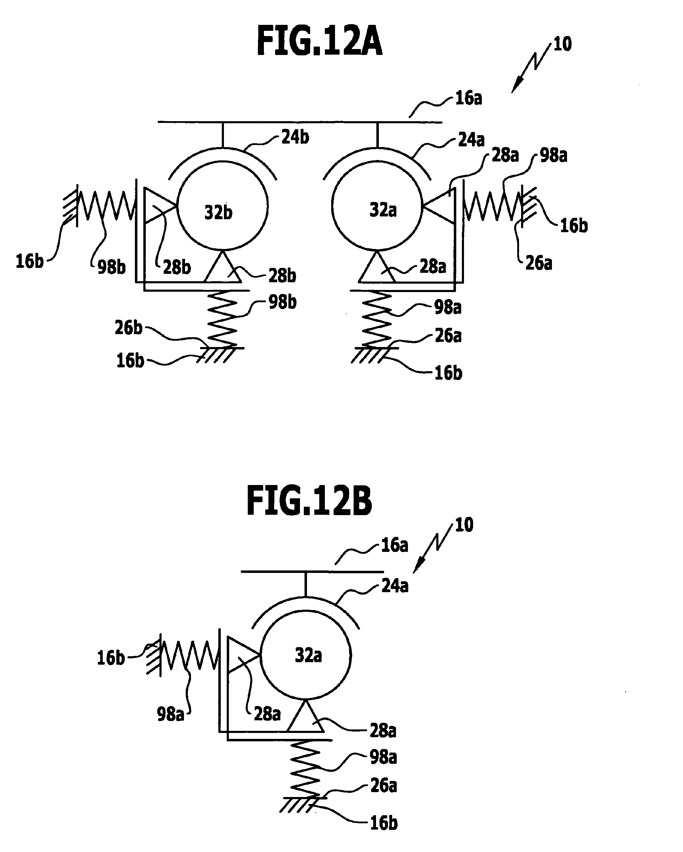

Der Unterschied zwischen den Bandscheibenprothesensystemen 10, wie sie in den Figuren 11A, 11B und 12A, 12B dargestellt ist, liegt in der Ausbildung der Rückstelleinrichtung 96. Bei den in den Figuren 11A, 11B dargestellten Bandscheibenprothesensystemen 10 findet eine Dämpfung nur in Folge einer Kompression in Richtung der Längsachse 128 statt, bei dem in den Figuren 12A, 12B dargestellten Bandscheibenprothesensystemen auch bei einer Bewegung des Kugelelements 32a, 32b in anteriorer Richtung.

Die dargestellten schematischen Varianten von Bandscheibenprothesensystemen sind rein beispielhaft. In jedem Fall ist bei allen Bandscheibenprothesensystemen entscheidend, dass zwei voneinander unabhängige Prothesenkomponente 20a und 20b ausgebildet werden, welche, wenn sie zwischen zwei Wirbelkörper 16a und 16b implantiert sind, ein gemeinsames Drehzentrum oder zumindest eine gemeinsame Dreh- oder Rotationsachse definieren können. Hierzu können die Kugelgelenke 32a, 32b in der beschriebenen und dargestellten Weise über entsprechende Gelenkelemente 28a, 28b und optional zum Einsatz kommende Rückstelleinrichtungen 96 in erforderlicher Weise in ihren Relativbewegungen aufeinander abgestimmt werden, um insbesondere ein gemeinsames Drehzentrum 56 derart zu definieren, dass die die Kugelgelenke 32a, 32b definierenden Gleitpaarungen stets flächig aneinander anliegen.

## Patentansprüche

1. Bandscheibenprothesensystem (10) zur Ausbildung einer künstlichen Bandscheibe umfassend eine erste Prothesenkomponente (20a) und eine von der ersten Prothesenkomponente unabhängige zweite Prothesenkomponente (20b), wobei jede der beiden Prothesenkomponenten (20a, 20b) ein erstes und ein zweites Wirbelkörperanlageelement (24a, 26a; 24b, 26b) zum Anlegen an benachbarte, ein Bandscheibenfach (12) einer Wirbelsäule (18) begrenzende Wirbelkörper (16a, 16b) und ein zwischen und relativ zu mindestens einem der ersten und zweiten Wirbelkörperanlageelementen (24a, 24b; 26a, 26b) beweglich gelagertes Gelenkelement (28a, 28b) umfasst, wobei jede Prothesenkomponente (20a, 20b) ein zwischen den ersten und zweiten Wirbelkörperanlageelementen (24a, 24b, 26a; 26b) beweglich gelagertes Kugelgelenk (32a; 32b) umfasst, **gekennzeichnet durch** eine Führungseinrichtung (52) zum Definieren eines übergeordneten, **durch** beide Prothesenkomponenten (20a, 20b) gemeinsam definierten Drehgelenks (54) zum gleichzeitigen Verdrehen der beiden ersten Wirbelkörperanlageelemente (24a, 24b) relativ zu den beiden zweiten Wirbelkörperanlageelementen (26a, 26b) um ein gemeinsames Drehzentrum (56), welches in einem Raumbereich (58) zwischen den Mittelpunkten (38a, 38b) der beiden Kugelgelenke (32a, 32b) liegt.

2. Bandscheibenprothesensystem nach Anspruch 1, **dadurch gekennzeichnet, dass** das Bandscheibenprothesensystem (10) derart ausgebildet ist, dass eine Position des Drehzentrums (56) im Raumbereich (58) in definierter Weise in Abhängigkeit einer Auslenkung des Bandscheibenprothesensystems (10) aus einer Grundstellung, in welcher es symmetrisch konfiguriert ist, veränderbar ist.

3. Bandscheibenprothesensystem nach einem der voranstehenden Ansprüche, **dadurch gekennzeichnet, dass** eine Verbindungslinie (60) der Mittelpunkte (38a, 38b) der beiden Kugelgelenke (32a, 32b) eine Flexions-/Extensionsachse des Bandscheibenprothesensystems (10) definiert.

4. Bandscheibenprothesensystem nach einem der voranstehenden Ansprüche, **dadurch gekennzeichnet, dass** das erste Wirbelkörperanlageelement (24a; 24b) eine erste Gelenkfläche (34a; 34b) definiert, dass das Gelenkelement (28a; 28b) eine zweite Gelenkfläche (36a; 36b) definiert, welche korrespondierend zur ersten Gelenkfläche (34a; 34b) ausgebildet ist, dass die zweite Gelenkfläche (36a; 36b) einen Teil einer Kugeloberfläche bildet und dass die erste Gelenkfläche (34a; 34b) einen Teil einer Hohlkugeloberfläche bildet.

5. Bandscheibenprothesensystem nach einem der vorstehenden Ansprüche, **dadurch gekennzeichnet, dass** die Führungseinrichtung (52) an jeder Prothesenkomponente (20a, 20b) jeweils eine Führungsfläche (64a, 64b) umfasst zum Führen einer Bewegung der beiden Kugelgelenke (32a, 32b) jeweils relativ zum zweiten Wirbelkörperanlageelement (26a, 26b).

6. Bandscheibenprothesensystem nach Anspruch 5, **dadurch gekennzeichnet, dass** die Führungsfläche (64a, 64b) ausgebildet ist zum Führen einer Bewegung des jeweiligen Gelenkelements (28a, 28b) relativ zum zweiten Wirbelkörperanlageelement (26a, 26b).

7. Bandscheibenprothesensystem nach Anspruch 6, **dadurch gekennzeichnet, dass** die Führungsfläche (64a, 64b) am Gelenkelement (28a, 28b) und/oder am zweiten Wirbelkörperanlageelement (26a, 26b) ausgebildet ist.

8. Bandscheibenprothesensystem nach einem der Ansprüche 5 bis 7, **dadurch gekennzeichnet, dass** das zweite Wirbelkörperanlageelement (26a, 26b) eine dritte Gelenkfläche (78a, 78b) definiert und dass das Gelenkelement (28a, 28b) eine vierte Gelenkfläche (76a, 76b) definiert, welche korrespondierend zur dritten Gelenkfläche (78a, 78b) ausgebildet ist, und dass die dritte und/oder die vierte Gelenkfläche (78a, 78b, 76a, 76b) die Führungsfläche (64a, 64b) bilden.

9. Bandscheibenprothesensystem nach einem der Ansprüche 5 bis 8, **dadurch gekennzeichnet, dass** die Führungsfläche (64a, 64b) eine Gelenkebene definiert.

10. Bandscheibenprothesensystem nach Anspruch 9, **dadurch gekennzeichnet, dass** das Gelenkelement (28a, 28b) relativ zum zweiten Wirbelkörperanlageelement (26a, 26b) parallel zur Gelenkebene verschiebbar und/oder um eine senkrecht zur Gelenkebene verlaufende Drehachse verdrehbar ist.

11. Bandscheibenprothesensystem nach Anspruch 9 oder 10, **dadurch gekennzeichnet, dass** die Gelenkebene relativ zu einer vom zweiten Wirbelkörperanlageelement (26a, 26b) definierten, in einer Grundstellung in Richtung auf das erste Wirbelkörperanlageelement (24a, 24b) weisende Längsachse um einen Neigungswinkel (66a, 66b) geneigt ist.

12. Bandscheibenprothesensystem nach einem der Ansprüche 8 bis 11, **dadurch gekennzeichnet, dass** die dritte Gelenkfläche (78a, 78b) in lateraler Richtung und in Richtung auf das erste Wirbelkörperanlageelement (24a, 24b) hin weist.

13. Bandscheibenprothesensystem nach einem der voranstehenden Ansprüche, **dadurch gekennzeichnet, dass** das Gelenkelement (28a, 28b) eine fünfte Gelenkfläche (80a, 80b) definiert und dass die fünfte Gelenkfläche (80a, 80b) direkt oder indirekt am zweiten Wirbelkörperanlageelement (26a, 26b) anliegt.

14. Bandscheibenprothesensystem nach einem der voranstehenden Ansprüche, **gekennzeichnet durch** eine Rückstelleinrichtung (96) zum Rückführen der beiden Kugelgelenke (32a, 32b) der Prothesenkomponenten (20a, 20b) von einer aus einer Grundstellung ausgelenkten Gelenkstellung zurück in die Grundstellung.

15. Bandscheibenprothesensystem nach einem der voranstehenden Ansprüche, **gekennzeichnet durch** eine Kugelgelenkanschlageinrichtung (40a; 40b) zum Begrenzen einer Relativbewegung der Gelenkelemente (28a; 28b) und der zugeordneten ersten Wirbelkörperanlageelemente (24a; 24b) relativ zueinander.

## Claims

1. An intervertebral disk prosthesis system (10) for forming an artificial intervertebral disk comprising a first prosthesis component (20a) and a second prosthesis component (20b) which is independent of the first prosthesis component, wherein each of the two prosthesis components (20a, 20b) comprises a first and a second vertebral body contacting element (24a, 24b; 26a, 26b) for placement on neighbouring vertebral bodies (16a, 16b) which bound an intervertebral disk space (12) of a spinal column (18) and a joint element (28a, 28b) that is mounted between and is moveable relative to at least one of the first and second vertebral body contacting elements (24a, 24b; 26a, 26b), wherein each prosthesis component (20a, 20b) comprises a ball joint (32a; 32b) that is mounted in moveable manner between the first and second vertebral body contacting elements (24a, 24b; 26a, 26b), **characterized by** a guidance device (52) for defining a superior swivel joint (54) that is defined by both prosthesis components (20a, 20b) together for simultaneously twisting the two first vertebral body contacting elements (24a, 24b) relative to the two second vertebral body contacting elements (26a, 26b) about a common centre of rotation (56) which is located in a spatial region (58) between the central points (38a, 38b) of the two ball joints (32a, 32b).

2. An intervertebral disk prosthesis system in accordance with Claim 1, **characterized in that** the intervertebral disk prosthesis system (10) is formed in such a manner that a position of the centre of rotation (56) in the spatial region (58) is changeable in a defined way in dependence on the deflection of the intervertebral disk prosthesis system (10) from the basic position in which it is symmetrically configured.

3. An intervertebral disk prosthesis system in accordance with any of the preceding Claims, **characterized in that** a connecting line (60) between the central points (38a, 38b) of the two ball joints (32a, 32b) defines a flexion/extension axis of the intervertebral disk prosthesis system (10).

4. An intervertebral disk prosthesis system in accordance with any of the preceding Claims, **characterized in that** the first vertebral body contacting element (24a; 24b) defines a first joint surface (34a; 34b), **in that** the joint element (28a; 28b) defines a second joint surface (36a; 36b) which is formed in correspondence with the first joint surface (34a; 34b), **in that** the second joint surface (36a; 36b) forms a part of a spherical surface and **in that** the first joint surface (34a; 34b) forms a part of a hollow ball surface.

5. An intervertebral disk prosthesis system in accordance with any of the preceding Claims, **characterized in that** the guidance device (52) on each prosthesis component (20a, 20b) comprises a respective guidance surface (64a, 64b) for guiding a movement of the two ball joints (32a, 32b) relative to the second vertebral body contacting element (26a, 26b).

6. An intervertebral disk prosthesis system in accordance with Claim 5, **characterized in that** the guidance surface (64a, 64b) is formed for guiding a movement of the respective joint element (28a, 28b) relative to the second vertebral body contacting element (26a, 26b).

7. An intervertebral disk prosthesis system in accordance with Claim 6, **characterized in that** the guidance surface (64a, 64b) is formed on the joint element (28a, 28b) and/or on the second vertebral body contacting element (26a, 26b)

8. An intervertebral disk prosthesis system in accordance with any of the Claims 5 to 7, **characterized in that** the second vertebral body contacting element (26a, 26b) defines a third joint surface (78a, 78b) and **in that** the joint element (28a, 28b) defines a fourth joint surface (76a, 76b) which is formed in correspondence with the third joint surface (78a, 78b), and **in that** the third and/or the fourth joint surface (78a, 78b, 76a, 76b) forms the guidance surface (64a, 64b).

9. An intervertebral disk prosthesis system in accordance with any of the Claims 5 to 8, **characterized in that** the guidance surface (64a, 64b) defines a joint plane.

10. An intervertebral disk prosthesis system in accordance with Claim 9, **characterized in that** the joint element (28a, 28b) is displaceable relative to the second vertebral body contacting element (26a, 26b) in parallel with the joint plane and/or can be rotated about an axis of rotation running perpendicularly relative to the joint plane.

11. An intervertebral disk prosthesis system in accordance with Claim 9, **characterized in that** the joint plane is inclined at an angle of inclination (66a, 66b) relative to a longitudinal axis which is oriented in the direction of the first vertebral body contacting element (24a, 24b) in the basic position and is defined by the second vertebral body contacting element (26a, 26b).

12. An intervertebral disk prosthesis system in accordance with any of the Claims 8 to 11, **characterized in that** the third joint surface (78a, 78b) is oriented in the lateral direction and in the direction of the first vertebral body contacting element (24a, 24b).

13. An intervertebral disk prosthesis system in accordance with any of the preceding Claims, **characterized in that** the joint element (28a, 28b) defines a fifth joint surface (80a, 80b) and **in that** the fifth joint surface (80a, 80b) rests directly or indirectly against the second vertebral body contacting element (26a, 26b).

14. An intervertebral disk prosthesis system in accordance with any of the preceding Claims, **characterized by** a re-setting device (96) for returning the two ball joints (32a, 32b) of the prosthesis components (20a, 20b) back into the basic position from a position into which the joint has been deflected from the basic position.

15. An intervertebral disk prosthesis system in accordance with any of the preceding Claims, **characterized by** a ball joint stop device (40a; 40b) for limiting any relative motion of the joint elements (28a; 28b) and the associated first vertebral body contacting elements (24a; 24b) relative to each other.

## Revendications

1. Système de prothèse de disque intervertébral (10) pour réaliser un disque intervertébral artificiel, comprenant un premier composant de prothèse (20a) et un deuxième composant de prothèse (20b) indépendant du premier composant de prothèse, système dans lequel chacun des deux composants de prothèse (20a, 20b) comprend un premier et un deuxième élément d'appui de vertèbre (24a, 26a ; 24b, 26b) destinés à s'appuyer sur des vertèbres (16a, 16b) voisines délimitant un espace de disque intervertébral (12) d'une colonne vertébrale (18), et comprend également un élément d'articulation (28a, 28b) monté entre le premier et le deuxième élément d' appui de vertèbre (24a, 24b ; 26a, 26b) et de manière mobile par rapport à au moins l'un d'eux, et système dans lequel chaque composant de prothèse (20a, 20b) comprend une articulation à rotule (32a ; 32b) montée mobile entre les premiers et deuxièmes éléments d'appui de vertèbre (24a, 24b ; 26a, 26b), **caractérisé par** un dispositif de guidage (52) destiné à définir une articulation de rotation (54) prévalente, définie en commun par les deux composants de prothèse (20a, 20b), pour la rotation simultanée des deux premiers éléments d'appui de vertèbre (24a, 24b) par rapport aux deux deuxièmes éléments d'appui de vertèbre (26a, 26b) autour d'un centre de rotation (56) commun, qui se situe dans une zone spatiale (58) entre les centres (38a, 38b) des deux articulations à rotule (32a, 32b).

2. Système de prothèse de disque intervertébral selon la revendication 1, **caractérisé en ce que** le système de prothèse de disque intervertébral (10) est conçu de façon telle, qu'il soit possible de faire varier de manière définie, une position du centre de rotation (56) dans la zone spatiale (58), en fonction d'une déviation du système de prothèse de disque intervertébral (10) hors d'une position de base dans laquelle il est de configuration symétrique.

3. Système de prothèse de disque intervertébral selon l'une des revendications précédentes, **caractérisé en ce qu'**une ligne de liaison (60) des centres (38a, 38b) des deux articulations à rotule (32a, 32b) définit un axe de flexion/extension du système de prothèse de disque intervertébral (10).

4. Système de prothèse de disque intervertébral selon l'une des revendications précédentes, **caractérisé en ce que** le premier élément d'appui de vertèbre (24a ; 24b) définit une première surface d'articulation (34a ; 34b), **en ce que** l'élément d'articulation (28a ; 28b) définit une deuxième surface d'articulation (36a ; 36b) réalisée de manière à correspondre à la première surface d'articulation (34a ; 34b), **en ce que** la deuxième surface d'articulation (36a ; 36b) forme une partie d'une surface sphérique, et **en ce que** la première surface d'articulation (34a ; 34b) forme une partie d'une surface sphérique creuse.

5. Système de prothèse de disque intervertébral selon l'une des revendications précédentes, **caractérisé en ce que** le dispositif de guidage (52) comprend sur chaque composant de prothèse (20a, 20b) respectivement une surface de guidage (64a, 64b) pour guider un mouvement des deux articulations à rotule (32a, 32b) respectivement par rapport au deuxième élément d'appui de vertèbre (26a, 26b).

6. Système de prothèse de disque intervertébral selon la revendication 5, **caractérisé en ce que** la surface de guidage (64a, 64b) est conçue pour guider un mouvement de l'élément d'articulation respectif (28a, 28b) par rapport au deuxième élément d'appui de vertèbre (26a, 26b).

7. Système de prothèse de disque intervertébral selon la revendication 6, **caractérisé en ce que** la surface de guidage (64a, 64b) est réalisée sur l'élément d'articulation (28a, 28b) et/ou sur le deuxième élément d'appui de vertèbre (26a, 26b).

8. Système de prothèse de disque intervertébral selon l'une des revendications 5 à 7, **caractérisé en ce que** le deuxième élément d'appui de vertèbre (26a, 26b) définit une troisième surface d'articulation (78a, 78b), et **en ce que** l'élément d'articulation (28a, 28b) définit une quatrième surface d'articulation (76a, 76b) réalisée de manière à correspondre à la troisième surface d'articulation (78a, 78b), et **en ce que** la troisième et/ou la quatrième surface d'articulation (78a, 78b, 76a, 76b) forment la surface de guidage (64a, 64b).

9. Système de prothèse de disque intervertébral selon l'une des revendications 5 à 8, **caractérisé en ce que** la surface de guidage (64a, 64b) définit un plan d'articulation.

10. Système de prothèse de disque intervertébral selon la revendication 9, **caractérisé en ce que** l'élément d'articulation (28a, 28b) peut coulisser, par rapport au deuxième élément d'appui de vertèbre (26a, 26b), parallèlement au plan d'articulation et/ou tourner autour d'un axe de rotation s'étendant perpendiculairement au plan d'articulation.

11. Système de prothèse de disque intervertébral selon la revendication 9 ou la revendication 10, **caractérisé en ce que** le plan d'articulation est incliné d'un angle d'inclinaison (66a, 66b) par rapport à un axe longitudinal défini par le deuxième élément d'appui de vertèbre (26a, 26b) et orienté en direction du premier élément d'appui de vertèbre (24a, 24b) dans une position de base.

12. Système de prothèse de disque intervertébral selon l'une des revendications 8 à 11, **caractérisé en ce que** la troisième surface d'articulation (78a, 78b) est orientée dans la direction latérale et en direction du premier élément d'appui de vertèbre (24a, 24b).

13. Système de prothèse de disque intervertébral selon l'une des revendications précédentes, **caractérisé en ce que** l'élément d'articulation (28a, 28b) définit une cinquième surface d'articulation (80a, 80b), et **en ce que** la cinquième surface d'articulation (80a, 80b) s'appuie directement ou indirectement sur le deuxième élément d'appui de vertèbre (26a, 26b).

14. Système de prothèse de disque intervertébral selon l'une des revendications précédentes, **caractérisé par** un dispositif de rappel (96) pour ramener à la position de base les deux articulations à rotule (32a, 32b) des composants de prothèse (20a, 20b), à partir d'une position d'articulation déviée hors de la position de base.

15. Système de prothèse de disque intervertébral selon l'une des revendications précédentes, **caractérisé par** un dispositif de butée d'articulation à rotule (40a ; 40b) destiné à limiter un mouvement relatif des éléments d'articulation (28a ; 28b) et des premiers éléments d'appui de vertèbre (24a ; 24b) les uns par rapport aux autres.
